# EUROPEAN PATENT APPLICATION

(11) **EP 4 570 823 A1**
(43) Date of publication of application: **18.06.2025**
(21) Application number: 23852040.7
(22) Date of filing: 26.09.2023
(51) Int. Cl.: C07K 16/18, C12N 1/21, G01N 33/577, A61K 39/395, A61P 25/28

(54) **IMPROVED ANTIBODY SPECIFICALLY BINDING TO AMYLOID-BETA OLIGOMERS**

(30) Priority: 09.08.2022 CN 202210949732
(71) Applicant: Shen Zhen Wisdom Biopharm Co., Ltd., Shenzhen, Guangdong 518067 (CN)
(72) Inventor: LIU, Ruitian, Shenzhen, Guangdong 518067 (CN); YU, Xiaolin, Shenzhen, Guangdong 518067 (CN); LU, Shuai, Shenzhen, Guangdong 518067 (CN); YANG, Jinju, Shenzhen, Guangdong 518067 (CN); ZHU, Jie, Shenzhen, Guangdong 518067 (CN)
(74) Representative: Høiberg P/S
(86) International application number: PCT/CN2023/121350
(87) International publication number: WO 2024/032822

(57) **Abstract**

The present invention relates to an improved antibody specifically binding to amyloid-β oligomers (AβOs). Specifically, the present invention relates to an improved form of the antibody W20. Compared with the antibody W20, the improved form of the antibody W20 has a significantly improved affinity to AβOs, and can more significantly inhibit the aggregation of Aβ and the AβOs-induced toxicity of nerve cells, more effectively improve the cognition and memory functions of an Alzheimer's disease model mouse, and reduce pathological changes in the brain of the mouse. The improved form of the antibody can specifically bind to oligomers of an amyloid-β, α-synuclein, mHTT and SOD 1, can inhibit the aggregation and cytotoxicity of various amyloids, and has a better potential for treating various amyloid diseases, such as Parkinson's disease, Huntington's disease, and amyotrophic lateral sclerosis, than the antibody W20. The improved form of the antibody can specifically bind to a highly toxic amyloid protein oligomer Aβo*3F, and has a better AD diagnosis value. The amino acid sequence of the antibody W20 is as shown in SEQ ID NO: 1.

## Description

### Technical Field

The present invention relates to antibodies specifically binding to amyloid-β oligomers (AβOs), in particular, to improved antibodies specifically binding to particular AβOs.

### Background Art

Alzheimer's disease (AD) is a chronic neurodegenerative disease often occurring in the elderly. Its clinical symptoms are mainly memory loss, cognitive impairment, and loss of self-care ability. AD has been listed as the fourth leading cause of death in humans, second only to cancer, heart disease, and stroke. Its incidence is apparently age-dependent. With the aging of the global population, the number of AD patients has increased year by year. According to statistics, there were about 50 million AD patients in the world in 2018, and it is estimated that the number of AD patients in the world may reach 130 million in 2050. AD medical expenses are huge. In 2015, the global cost of treatment and care for AD patients was about 818 billion US dollars, and it is expected to increase to 2 trillion US dollars in 2030. In the past decade, the world has invested about 110 billion US dollars in the research and development of AD therapeutic drugs, but the huge investment of manpower and material resources has not yet led to the launch of specific therapeutic drugs, and hundreds of therapeutic drugs have failed in clinical trials. The development of drugs targeting the pathogenesis of AD has become a research hotspot that many international pharmaceutical companies and research institutions are competing for.

AD is a neurodegenerative disease in the elderly caused by the toxic oligomers formed by the aggregation of amyloid protein Aβ and microtubule-associated protein tau, mainly characterized by memory loss, the formation of senile plaques in the brains, and the formation of neural entanglements in neurons. It is generally believed that the most important factor causing the occurrence and development of AD is the most cytotoxic Aβ oligomers, rather than Aβ monomers and fibers. The level of Aβ oligomers in the brains of AD patients is highly correlated with the occurrence, development, and severity of AD. Aβ oligomers have a high affinity for synapses. They can trigger the redistribution of key synaptic proteins, induce excessive activation of synaptic-related glutamate receptors, and can also damage nerve cell membranes, disrupt synaptic Ca²⁺ homeostasis, and thereby increase oxidative stress and mitochondrial damage in cells. Aβ oligomers bind to synapses to activate the complement system, prompting microglia to over-prune and phagocytose synapses, destroying the structure of synapses, inducing synaptic dysfunction, causing synaptic damage and excessive loss, and leading to cognitive decline. Therefore, targeting Aβ oligomers and inhibiting the level of Aβ oligomers and their induced neurotoxicity is one of the ideal strategies for treating AD for the cause. In contrast, treatment regimens targeting Aβ monomers, especially immunotherapy targeting Aβ monomers, can cause adverse reactions such as inflammation and synaptic loss, leading to repeated failures in clinical trials. Clearing Aβ and reducing the level of Aβ oligomers through immunotherapy strategies have always been the focus of research in the field of AD treatment. Recently, dozens of antibodies and vaccines targeting Aβ have entered the clinical trial stage. For example, the first-generation vaccine AN1792 targeting Aβ fibers, and antibodies targeting Aβ monomers or fibers such as bapineuzumab (anti-Aβ1-5), solanezumab (anti-Aβ13-28), gantenerumab (anti-Aβ1-11) and ponezumab (anti-Aβ C-terminus), etc. Although these preparations have shown good therapeutic effects in the animal experiment stage, significantly improving the cognitive level of AD transgenic animals, reducing the amount of senile plaques and other pathological changes in the animal brains, in AD clinical trials, due to poor therapeutic effects or serious side effects, few have passed the Phase III clinical trial so far. In June 2021, the US FDA approved the Aduhelm (aducanumab) antibody for marketing through an accelerated approval program. Aducanumab can specifically recognize Aβ oligomers and has shown certain efficacy in clinical trials, but this antibody still has adverse reactions such as brain edema and inflammation, and the efficacy of this antibody is also controversial.

Many neurodegenerative diseases are characterized by abnormal aggregation of amyloid proteins, such as Aβ, tau, α-synuclein, mHTT and SOD1. The oligomers formed by the aggregation of these protein monomers are key pathogenic factors for the occurrence and development of AD, Parkinson's disease (PD), Huntington's disease (HD), amyotrophic lateral sclerosis (ALS), etc., and are also effective targets for the diagnosis and treatment of such diseases. Oligomers and fibers of different amyloid proteins are deposited in different parts of the brain, damaging nerve cells in the corresponding areas, leading to cognitive and behavioral disorders. Although these amyloid proteins have different primary sequences, their oligomers formed by aggregation can form similar spatial structures and have similar toxicity mechanisms. Therefore, therapeutic strategies targeting amyloid toxic oligomers are of great significance for the diagnosis and treatment of various neurodegenerative diseases. Additionally, studies have shown that various amyloid proteins have a synergistic promoting effect in amyloid diseases. The aggregation of a certain amyloid protein and the occurrence and development of the disease will promote or induce the aggregation of other amyloid proteins and participate in the pathological process of the diseases. For example, clinical studies have found that in addition to the deposition of Aβ plaques and neurofibrillary entanglement caused by tau protein, 50% of AD patients also have apparent Lewy bodies (LB) produced by the aggregation and deposition of α-synuclein in their brains. Compared with AD patients without LB deposition, these patients have more severe pathological characteristics and decreased cognitive function. Transgenic animal experiments have shown that Aβ, tau, and α-synuclein can synergize with each other, promote each other's aggregation and deposition, and accelerate pathological changes and cognitive impairment in the brains of transgenic mice. Therefore, treatments targeting only one amyloid protein may not be enough to cure amyloid disease. Preparations that specifically target the structural features shared by multiple amyloid protein oligomers without relying on their primary amino acid sequences will become an ideal strategy for treating one or more amyloid diseases.

Since 2005, the inventors have conducted research on amyloid protein oligomer-specific antibodies and were the first in the world to screen and obtain a fully human single-chain antibody W20 that can recognize a variety of amyloid protein oligomers (Chinese patent No. CN101463082A). This antibody can bind to oligomers formed by, for example, Aβ, α-synuclein, mHTT, and SOD1 that cause various amyloid protein diseases such as AD, PD, HD and ALS, but not to monomers and fibers of each amyloid protein. This antibody can also inhibit the aggregation and cytotoxicity of each amyloid protein; improve the cognitive function and behavioral coordination ability of relevant disease model mice, and reduce the neuropathological changes in mice (Biochimica etBiophysicaActa 2011, 1814, 1703-1712; AD: Current Alzheimer Research, 2014, 11, 69-78; PD&HD: Scientific Reports, 2016, 6, 36631; ALS: International Immunopharmacology 2018, 65, 413-421).

To date, there is still an urgent need in the art to further enhance the drugability of amyloid protein oligomer-specific antibodies and to develop antibody drugs that can effectively treat and/or prevent neurodegenerative diseases.

### Summary of the invention

One aspect of the present invention relates to an improved amyloid protein oligomer-specific antibody or an antigen-binding fragment thereof, having amino acid substitutions at one or more positions selected from the group consisting of amino acid residue positions 226, 227 and 228 relative to the antibody W20, of which the amino acid sequence is shown in SEQ ID No. 1, and the amino acid position numbering of the antibody being numbered according to the Kabat numbering system.

In some embodiments, the amino acid residue at position 226 of the improved amyloid protein oligomer-specific antibody or antigen-binding fragment thereof is substituted with a nonpolar, hydrophobic amino acid having properties similar to those of alanine, preferably, substituted with glycine, valine, leucine, isoleucine, phenylalanine, tryptophan or proline, more preferably, substituted with glycine, phenylalanine or tryptophan.

In some embodiments, the amino acid residue at position 227 of the improved amyloid protein oligomer-specific antibody or antigen-binding fragment thereof is substituted with a hydrophobic amino acid having properties similar to those of valine, preferably, substituted with phenylalanine, tryptophan, tyrosine, alanine, leucine or isoleucine, more preferably, substituted with phenylalanine, isoleucine or leucine.

In some embodiments, the amino acid residue at position 228 of the improved amyloid protein oligomer-specific antibody or antigen-binding fragment thereof is substituted with an aliphatic amino acid having a structure similar to that of arginine, preferably, substituted with alanine, valine, leucine, isoleucine, methionine, aspartic acid, glutamic acid, lysine, glycine, serine, threonine, cysteine, asparagine or glutamine, more preferably, substituted with lysine, asparagine or glutamine.

In some embodiments, the amino acid residues at positions 226-228 of the improved amyloid protein oligomer-specific antibody or antigen-binding fragment thereof are substituted with AVR, GSR, WVR, FER, NFR or VRR, respectively.

In some embodiments, the amino acid residues at positions 224 and 225 of the improved amyloid protein oligomer-specific antibody or antigen-binding fragment thereof are substituted with glutamine and threonine, respectively.

In some embodiments, the improved amyloid protein oligomer-specific antibodies or antigen-binding fragments thereof can all specifically bind to the Aβo*3F oligomers.

In some embodiments, the improved amyloid protein oligomer-specific antibody or antigen-binding fragment thereof is an antigen-binding fragment, preferably, an antigen-binding fragment selected from the group consisting of scFv, F(ab')₂, Fab', Fab, Fd, Fv, bispecific antibody, camel antibody, CDR and antibody minimal recognition unit (dAb), more preferably, an antigen-binding fragment selected from the group consisting of scFv, F(ab')₂, Fab', and Fab. In some embodiments, the improved amyloid protein oligomer-specific antibody or antigen-binding fragment thereof is scFv or F(ab')₂.

In some embodiments, the improved amyloid protein oligomer-specific antibody or antigen-binding fragment thereof is a monoclonal antibody, a chimeric antibody, a humanized antibody, or a fully human antibody, preferably, the antibody is selected from the group consisting of IgG, IgM, IgA, IgD, IgE and their subtypes, more preferably, the antibody is selected from the group consisting of subtypes IgG1-4, more preferably, the antibody is IgG 4 subtype. In some embodiments, the heavy chain amino acid sequence of the antibody is shown in any one of SEQ ID Nos. 18-21, and/or the light chain amino acid sequence of the antibody is shown in SEQ ID No. 17. In some embodiments, the heavy chain constant region amino acid sequence of the antibody is shown in any one of SEQ ID No s. 23-26, and/or the light chain constant region amino acid sequence of the antibody is shown in SEQ ID No. 22.

In some embodiments, the improved amyloid protein oligomer-specific antibody or antigen-binding fragment thereof is a scFv of which the amino acid sequence is shown in any one of SEQ ID Nos. 5, 4 and 6-9.

In some embodiments, the amino acid sequence of CDR3 of the light chain of the improved amyloid protein oligomer-specific antibody or antigen-binding fragment thereof is as shown in SEQ ID No. 15.

In some embodiments, the amino acid residue at position 98 of the improved amyloid protein oligomer-specific antibody or antigen-binding fragment thereof relative to the antibody 3F is substituted with arginine. In some embodiments, the heavy chain amino acid sequence of the improved amyloid protein oligomer-specific antibody or antigen-binding fragment thereof is as shown in any one of SEQ ID Nos. 33-36, and the light chain amino acid sequence thereof is as described above. In some embodiments, the improved amyloid protein oligomer-specific antibody or antigen-binding fragment thereof is scFv of which the amino acid sequence is as shown in SEQ ID No. 27.

In some embodiments, the present invention relates to a variant of the improved amyloid protein oligomer-specific antibody or antigen-binding fragment thereof as described above, which has a substitution, insertion, or deletion of one or more amino acid residues relative to the improved amyloid protein oligomer-specific antibody or antigen-binding fragment thereof as described above, such as a substitution, insertion, or deletion of 1, 2, 3, 4, 5, 10, 15, 20, 25, 30 or more amino acid residues, while still substantially maintains one, more or all biological activities of the improved amyloid protein oligomer-specific antibody or antigen-binding fragment thereof as described above. In some embodiments, the substitution of the amino acid residue is a substitution of a conservative amino acid residue. In some embodiments, the present invention relates to a variant of the improved amyloid protein oligomer-specific antibody or antigen-binding fragment thereof as described above, which has at least 85% identity with the improved amyloid protein oligomer-specific antibody or antigen-binding fragment thereof as described above, such as at least 90%, 91%, 92%, 93%, 94%, 95%, 96%, 97%, 98%, 99%, 99.1%, 99.3%, 99.6% or higher identity, while still substantially maintains one, more or all biological activities of the improved amyloid protein oligomer-specific antibody or antigen-binding fragment thereof as described above. It is known to those skilled in the art that the above identity is calculated on basis of individual amino acid residues and therefore, the above numerical values are only approximate values, and those skilled in the art can accurately calculate the corresponding identity percentage according to the number of substitutions, insertions or deletions of the amino acid residues that actually occur.

Another aspect of the present invention relates to an isolated nucleic acid molecule selected from the group consisting of:
(1) DNA or RNA encoding the improved amyloid protein oligomer-specific antibody or antigen-binding fragment thereof as described above;
(2) a nucleic acid molecule completely complementary to the DNA or RNA defined in item (1).

A further aspect of the present invention relates to an expression vector comprising an operably linked nucleic acid molecule as described above.

Another aspect of the present invention relates to a host cell comprising the nucleic acid molecule or expression vector as described above.

Another aspect of the present invention relates to a composition comprising the improved amyloid protein oligomer-specific antibody or antigen-binding fragment thereof, nucleic acid molecule, expression vector or host cell as described above, and one or more of pharmaceutically acceptable carriers, diluents, and excipients.

Another aspect of the present invention relates to a method for producing the improved amyloid protein oligomer-specific antibody or antigen-binding fragment thereof as described above, comprising the steps of:
incubating the host cells described above under culture conditions suitable for the expression of the improved amyloid protein oligomer-specific antibody or antigen-binding fragment thereof, and optionally, isolating and purifying the obtained product.

Yet another aspect of the present invention relates to a composition comprising the improved amyloid protein oligomer-specific antibody or antigen-binding fragment thereof, the nucleic acid molecule, the expression vector, the host cell, as described above, or the improved amyloid protein oligomer-specific antibody or antigen-binding fragment thereof, the nucleic acid molecule, the expression vector, the host cell obtained by the method as described above, and an additional neurodegenerative disease therapeutic agent, preferably, the additional neurodegenerative disease therapeutic agent is selected from the group consisting of acetylcholinesterase inhibitors such as donepezil, galantamine, carbastine, etc., and aspartate receptor antagonists such as memantine, etc.

Another aspect of the present invention relates to the use of the improved amyloid protein oligomer-specific antibody or antigen-binding fragment thereof, nucleic acid molecule, expression vector, host cell or composition as described above in the manufacture of a medicament for inhibiting the aggregation of Aβ and/or Aβ oligomer-induced cytotoxicity in a subject, or for treating and/or preventing a neurodegenerative disease in a subject, or for diagnosing whether a subject suffers from a neurodegenerative disease.

In some embodiments, the present invention relates to the improved amyloid protein oligomer-specific antibody or antigen-binding fragment thereof, nucleic acid molecule, expression vector, host cell or composition as described above for use in inhibiting Aβ aggregation and/or Aβ oligomer-induced cytotoxicity in a subject, or in treating and/or preventing a neurodegenerative disease in a subject, or in diagnosing whether a subject suffers from a neurodegenerative disease.

Another aspect of the present invention relates to a method for inhibiting Aβ aggregation and/or Aβ oligomer-induced cytotoxicity in a subject, or for treating and/or preventing a neurodegenerative disease in a subject, or for diagnosing whether a subject suffers from a neurodegenerative disease, comprising a step of administering to the subject or the subject's cells a therapeutically effective amount of the improved amyloid protein oligomer-specific antibody or antigen-binding fragment thereof, the nucleic acid molecule, the expression vector, the host cell or the composition as described above.

In some embodiments, the neurodegenerative disease is selected from the group consisting of Alzheimer's disease, Parkinson's disease, Huntington's disease, amyotrophic lateral sclerosis, frontotemporal dementia, and spinocerebellar ataxia, preferably, the neurodegenerative disease is selected from the group consisting of Alzheimer's disease, Parkinson's disease, Huntington's disease, and amyotrophic lateral sclerosis.

Another aspect of the present invention relates to the use of the improved amyloid protein oligomer-specific antibody or antigen-binding fragment thereof, nucleic acid molecule, expression vector, host cell or composition in the manufacture of an agent for diagnosing the presence and/or level of toxic forms of amyloid in a sample from a subject.

Another aspect of the present invention relates to the use of the improved amyloid protein oligomer-specific antibody or antigen-binding fragment thereof, nucleic acid molecule, expression vector, host cell or composition as described above in the manufacture of a medicament for specifically binding to the highly toxic amyloid protein oligomer Aβo*3F and inhibiting its neurotoxicity in a subject, wherein the highly toxic amyloid protein oligomer Aβo*3F is separated from an Aβ oligomer mixture by immunoprecipitation with the antibody 3F, typically characterized as an Aβ oligomer with high molecular weight, with a molecular weight of approximately 588 kDa and a diameter of approximately 10 nm based on size exclusion chromatography (SEC) analysis.

In some embodiments, the amyloid protein is selected from the group consisting of amyloid-β protein, α-synuclein, mHTT, and SOD1.

In other words, in order to enhance the drugability of the antibody, the present invention has carried out a mature mutation transformation on the antibody W20, with the main mutation sites locating in the light chain CDR3 region, for example, the QTHRP sequence is mutated to the NSAVR sequence. The results of molecular simulation experiments show that the improved antibodies of the present invention, such as the antibody 3F, can recognize a specific structure on the oligomers formed by aggregation of the amyloid protein. Furthermore, the improved antibodies, such as the antibody 3F, have significantly improved affinity for binding to the antigen Aβ oligomers, and the half-life thereof *in vivo* is also significantly prolonged. The antibody 3F can inhibit the aggregation and cytotoxicity of various amyloid proteins such as Aβ, α-synuclein, and mHTT *in vitro;* nasal administration at a dose level of µg for 20 days can significantly improve the memory of AD transgenic mice and reduce pathological changes and inflammatory reactions in the brain. The results of acute toxicity tests using antibodies 1000 times greater than the therapeutic dose showed that the animals were well tolerated and no pathological changes were observed in organs and tissues. Therefore, the antibody 3F has obvious therapeutic effects, good safety, good stability, and good drugability.

More importantly, the Aβ oligomers specifically recognized by the antibody are the oligomers having extremely strong toxicity, which are the main toxic components in the mixture of Aβ oligomers, have strong pathogenic effects, and play a key role in the occurrence and development of AD. The highly toxic oligomers recognized by the antibody 3F exist in the CSF, blood and/or brain tissues of AD patients and AD-derived MCI patients, and their levels show extremely significant differences in the CSF, blood and/or brain of three groups of people: AD patients, MCI patients and healthy elderly people. Therefore, AD patients, MCI patients and healthy elderly people can be accurately distinguished. This toxic oligomer also exists in AD transgenic mice and is directly related to the onset of AD transgenic mice. In the present invention, the highly toxic Aβ oligomer recognized by the antibody 3F is called AβO*3F, which can be separated from the Aβ oligomer mixture by immunoprecipitation (with the antibody 3F). Its typical characteristic is that it belongs to Aβoligomers with a high molecular weight. Based on molecular exclusion chromatography (SEC) analysis, its molecular weight is about 588 kDa and its diameter is about 10 nm. It has a strong toxic effect on neurons, which is more than 200 times stronger than that of the Aβ oligomer mixture. The Aβo*3F can activate microglia and/or astrocytes to secrete a large number of inflammatory factors. More importantly, the present invention prepared the Aβo*3F *in vitro* and the Aβo*3F prepared *in vitro* is consistent in composition with the product immunoprecipitated from the cerebrospinal fluid or plasma of AD patients using the antibody 3F, and has the same physicochemical properties and functions. Although some prior art monoclonal antibodies such as lecanemab also target Aβ aggregates, the Aβ aggregates targeted thereby are different from the Aβ aggregates targeted by the improved antibodies of the present invention such as the antibody 3F, which specifically targets the highly neurotoxic oligomer Aβo*3F as described above.

In particular, the improved antibodies of the present invention, such as the antibody 3F, have the following advantages and characteristics.
1) Compared with the antibody W20, the affinity between the antibody 3F and the highly toxic amyloid protein oligomer Aβo* 3F is significantly improved, and the antibody 3F can better inhibit the aggregation and cytotoxicity of amyloid proteins and has better therapeutic potential.
2) The highly toxic amyloid protein oligomer Aβo*3F specifically bound by the antibody 3F is present in the cerebrospinal fluid (CSF), blood and/or brain tissues of AD patients and AD-derived mild cognitive impairment (MCI) patients, and its levels in the CSF, blood and/or brain of the following three groups of people: AD patients, MCI patients and healthy elderly people, show extremely significant differences. It is an oligomer having super toxicity and is the main toxic component in the Aβ oligomer mixture. It has a strong pathogenic effect and plays a key role in the occurrence and development of AD. Therefore, the Aβo*3F can be used as a target for the diagnosis, prevention and/or treatment of MCI and/or AD in a subject. The antibody 3F targeting the Aβo*3F has good application value in the diagnosis and treatment of AD.
3) The antibody 3F specifically binds to the highly toxic amyloid protein oligomer Aβo*3F, which is a key pathogenic factor, does not recognize monomers and fibers of amyloid proteins, and does not cause autoimmune responses. Since monomers of amyloid proteins, such as Aβ and α-synuclein, have normal physiological functions in the body, antibodies binding to these monomers are prone to cause autoimmune responses and reduce the effective concentration of the targets targeted by the antibodies in the body. This is also one of the main reasons for that many antibodies and vaccine drugs have failed in clinical trials. In addition, most of the dozens of antibodies and vaccines targeting Aβ that have entered the clinical trial stage have poor therapeutic effects or serious adverse reactions. Studies have shown that since many antibodies target Aβ monomers and the antigen-antibody complexes thereof will trigger inflammatory reactions and cause synaptic loss, resulting in the failure of Aβ-targeted immunotherapy for AD. In contrast, the antibody 3F can overcome this side effect and achieve a better AD treatment effect.
4) The antibody 3F can bind to a variety of amyloid proteins with different primary sequences, reduce their toxicity and promote their clearance. Amyloid proteins with different primary structures can form similar structures when aggregated, for example, they all are rich in β sheet structures, and can all bind to thioflavin (ThT) and Congo red, etc., which constitutes the basis for the antibody 3F to bind to a variety of amyloid protein oligomers. The antibody 3F can specifically bind to oligomers of Aβ, α-synuclein, mHTT and SOD 1, and inhibit the aggregation and cytotoxicity of each amyloid protein. Compared with the antibody W20, the antibody 3F has a more significant therapeutic effect on model animals such as AD, PD, HD, ALS, etc., and has greater application potential.
5) The therapeutic dosage of the antibody 3F is small, while the therapeutic effect thereof is obvious, the therapeutic mechanism thereof is clear, the safety thereof is high, and the properties thereof are stable.
6) Since the Aβo*3F is an oligomer form formed by the aggregation of Aβ monomers, all antibodies targeting Aβ monomers may bind to the Aβo*3F. However, compared with the antibodies of the present invention, such as the antibody 3F specifically recognizing and binding to the Aβo*3F, antibodies targeting Aβ monomers in the prior art can also bind to various forms such as Aβ monomers, oligomers, and fibers. Therefore, the chance of binding to the highly neurotoxic oligomer Aβo*3F of the present invention thereof is greatly reduced, and accordingly, its efficacy is also weakened, resulting in a decrease in the AD treatment effect.

### BRIEF DESCRIPTION OF THE DRAWINGS

Figure 1 shows the polyclonal ELISA of the phage antibody library.
Figure 2 shows the monoclonal ELISA of the phages.
Figure 3 shows the structural molecular simulation of the single-chain antibody W20 and its key amino acid residues.
Figure 4 shows that the antibody 3F specifically binds to Aβ oligomers. Dot blot experiments were performed to detect the binding ability of the antibody 3F to Aβ42 monomers and aggregates.
Figure 5 shows that the antibody 3F can significantly inhibit Aβ aggregation and Aβ oligomer-induced cytotoxicity:
   A. Thioflavin T (ThT) assay was used to detect the inhibitory effects of the antibody 3F and the antibody W20 on Aβ aggregation;
   B. MTT assay was used to detect the inhibitory effects of the antibody 3F and the antibody W20 on Aβ oligomer-induced nerve cell cytotoxicity.
Figure 6 shows that the antibody 3F can significantly improve the cognitive function of AD transgenic mice:
   A. The latency of each group of mice to find the platform during the training period in the water maze experiment;
   B. The number of times the mouse crossed the platform during the exploration period after the platform was removed in the water maze experiment;
   C. The time that each group of mice stayed in the new arm in the Y maze experiment.

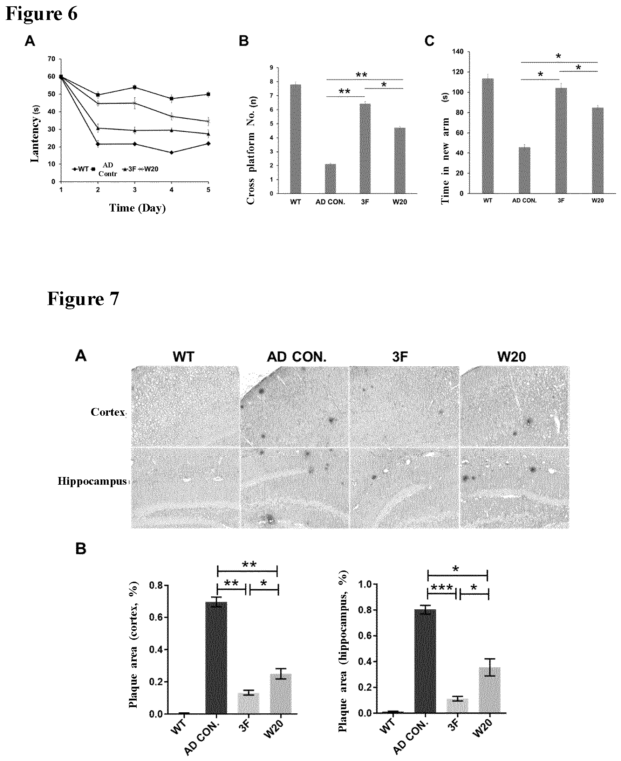

Figure 7 shows that the antibody 3F can significantly reduce the level of Aβ plaques in the brains of AD transgenic mice:
   A. APP/PS 1 mouse brain slices were immunostained using the antibody 4G8 to detect the level of senile plaques in the mouse brain;
   B. Quantitative statistics of the senile plaque area in the cortex and hippocampus of mice.

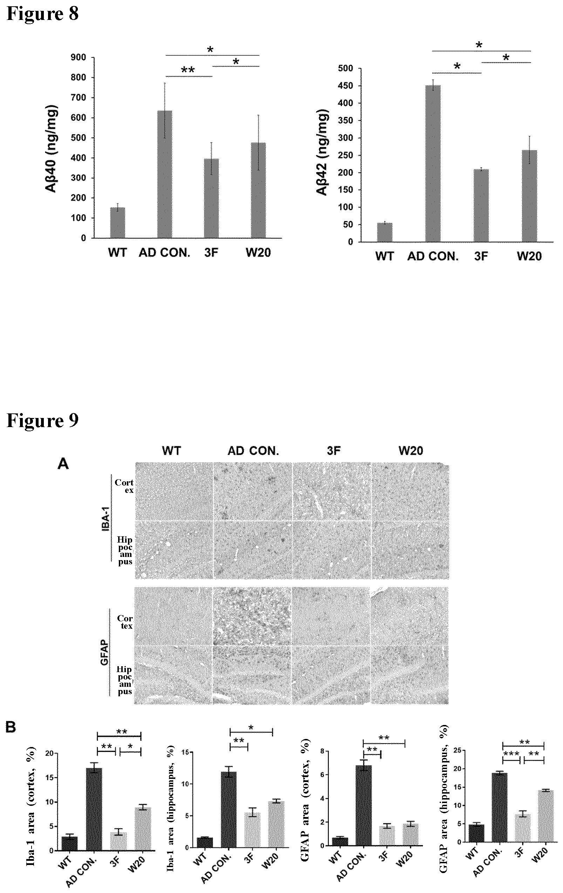

Figure 8 shows that the antibody 3F can significantly reduce the level of Aβ in the brains of AD transgenic mice. The ELISA assay was used to detect the content of Aβ40/42 in the brain homogenate of APP/PS 1 mice.
Figure 9 shows that the antibody 3F can significantly reduce the activation level of glial cells in the brains of AD transgenic mice:
   A. The activation of microglia and astrocytes in the brains of APP/PS 1 mice was detected by Iba-1 immunostaining and GFAP immunostaining;
   B. Quantitative statistics of Iba-1 and GFAP positive staining areas in the cortex and hippocampus of mouse brain.

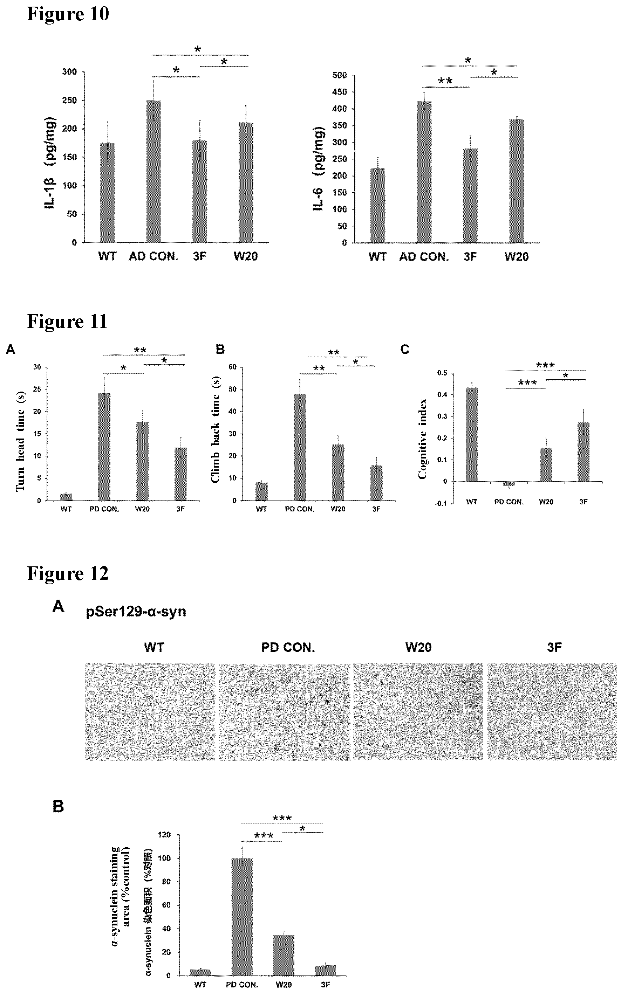

Figure 10 shows that the antibody 3F can significantly reduce the level of inflammatory factors in the brains of AD transgenic mice. The ELISA assay was used to detect the content of IL-1β and IL-6 in the brain homogenate of APP/PS1 mice.
Figure 11 shows that the antibody 3F can significantly improve the behavioral coordination ability and cognitive function of PD transgenic mice:
   A. The time taken by each group of mice to turn their heads in the pole climbing test;
   B. The time taken by each group of mice to climb into the cage in the pole climbing test;
   C. Cognitive index of each group of mice in the novel object recognition experiment.
Figure 12 shows that the antibody 3F can significantly reduce the level of α-synuclein in the brains of PD transgenic mice:
   A. The brain slices of A53T α-synuclein transgenic mice were immunostained with an anti-pSer129-α-syn antibody to detect the pathological α-synuclein level in the mouse brain;
   B. Quantitative statistics of pSer129-α-syn positive staining area in the mouse brainstem region.

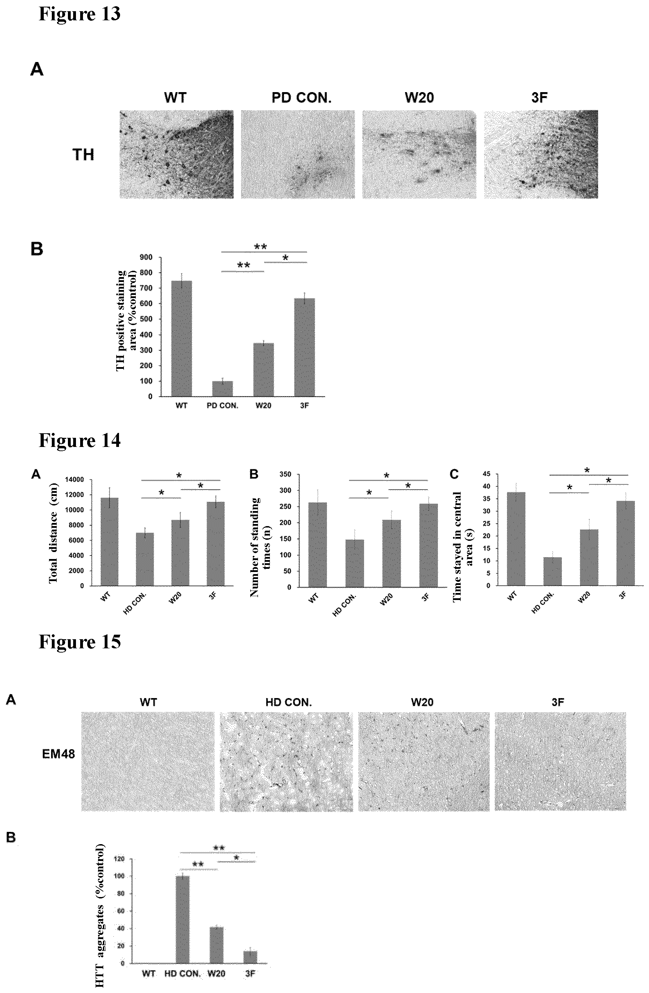

Figure 13 shows that the antibody 3F can significantly increase the level of tyrosine hydro xylase in the brains of PD transgenic mice:
   A. The brain slices of A53T α-synuclein transgenic mice were immunostained with an anti-Tyrosine hydroxylase (TH) antibody to detect TH levels in the mouse brain;
   B. Quantitative statistics of TH-positive staining area in the mouse brainstem region.
Figure 14 shows that the antibody 3F can significantly improve the spontaneous activity ability and anxiety behavior of HD transgenic mice: statistical analysis of the total distance traveled (A), the number of standing times in all areas (B), and the time stayed in the central area (C) of each group of mice in the open field test.
Figure 15 shows that the antibody 3F can significantly reduce the level of mHTT aggregates in the brains of HD transgenic mice:
   A. R6/2 mouse brain slices were immunostained with EM48 antibody to detect the level of mHTT aggregates in the mouse brain;
   B. Quantitative statistics of EM48 positive staining area in mouse brain.

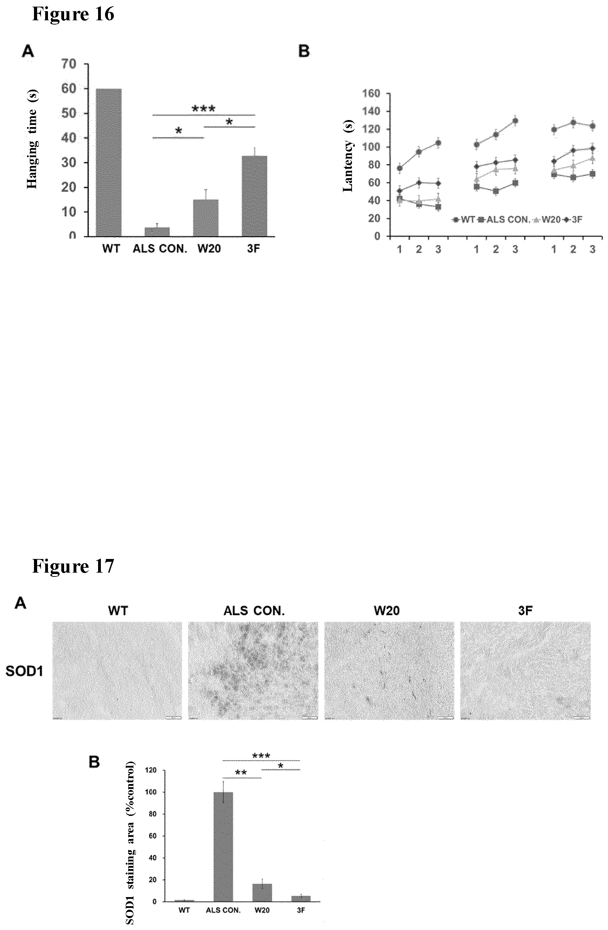

Figure 16 shows that the antibody 3F can significantly improve the motor function of ALS transgenic mice:
   A. The duration of stay of each group of mice in the hanging experiment;
   B. The duration of each group of mice on the rotarod in the rotarod test.
Figure 17 shows that the antibody 3F can significantly reduce the level of SOD1 aggregates in the brainstem of ALS transgenic mice:
   A. SOD1-G93A mouse brain slices were immunostained with an anti-SOD1 antibody to detect the level of SOD1 aggregates in the mouse brain;
   B. Quantitative statistics of SOD1-positive staining area in mouse brainstem.

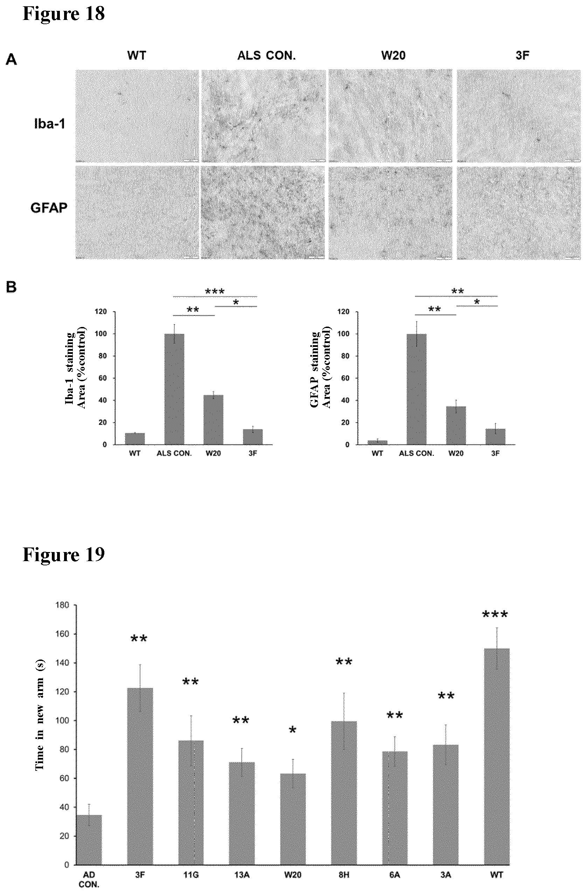

Figure 18 shows that the antibody 3F can significantly reduce the activation level of glial cells in the brains of ALS transgenic mice:
   A. The activation level of microglia and astrocytes in the brains of SOD1-G93A mice was detected by Iba-1 immunostaining and GFAP immunostaining;
   B. Quantitative statistics of Iba-1 and GFAP positive staining areas in mouse brain.
Figure 19 shows that the antibody can improve the cognitive function of AD transgenic mice. The time that each group of APP/PS 1 mice stayed in the new arm in the Y-maze test.

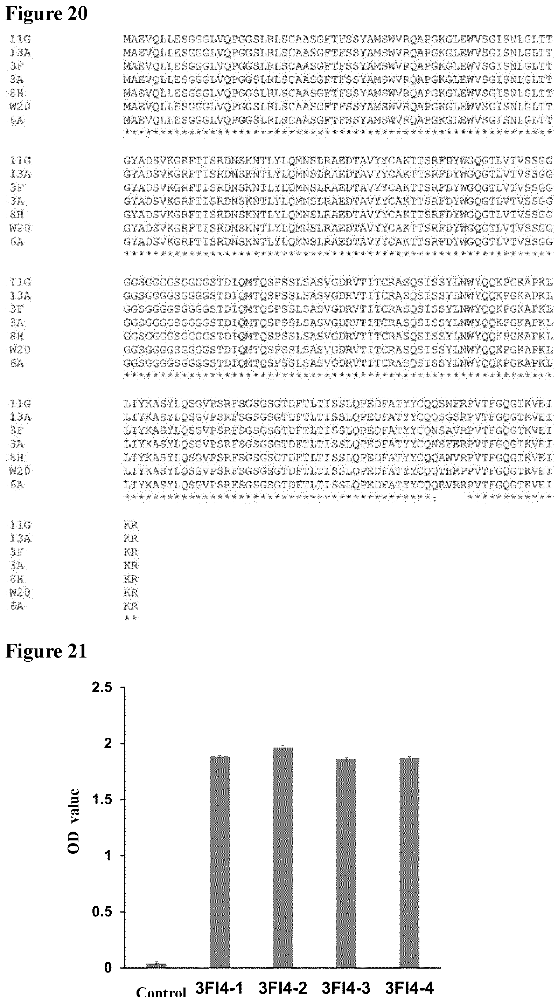

Figure 20 shows the results of alignment of the sequences of the positive clones with the sequence of the antibody W20.
Figure 21 shows that the antibody 3FI4 (IgG4 subtype of the antibody A16) can specifically bind to Aβ oligomers.

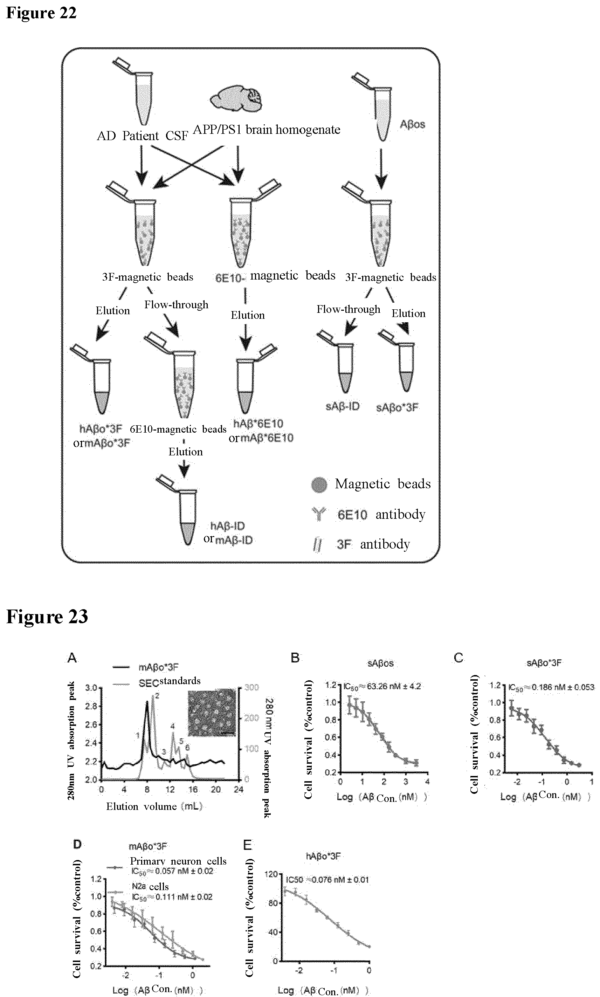

Figure 22 shows a schematic diagram of the immunoprecipitation preparation of the Aβo*3F, Aβ*6E10 and Aβ-ID.
Figure 23 shows the molecular weight, morphology characterization and cytotoxicity of the Aβo*3F:
   A: Size exclusion chromatography (SEC) analysis of the molecular weight of mAβo* 3F; SEC analysis of mAβo*3F and SEC standards (Superdex 200 10/300), SEC standards: 1. Thyroglobulin (669 kDa), 2. Ferritin (440 kDa), 3. Aldolase (158 kDa), 4. Conalbumin (75 kDa), 5. Ovalbumin (44 kDa), 6. Carbonic anhydrase (29 kDa);
   B: IC₅₀ values of sAβos against N2a cells for cytotoxicity;
   C: IC₅₀ values of sAβo*3F against N2a cells for cytotoxicity;
   D: IC₅₀ values of mAβo*3F against N2a cells and primary neurons for cytotoxicity;
   E: IC₅₀ values of hAβo*3F against primary neurons for cytotoxicity.

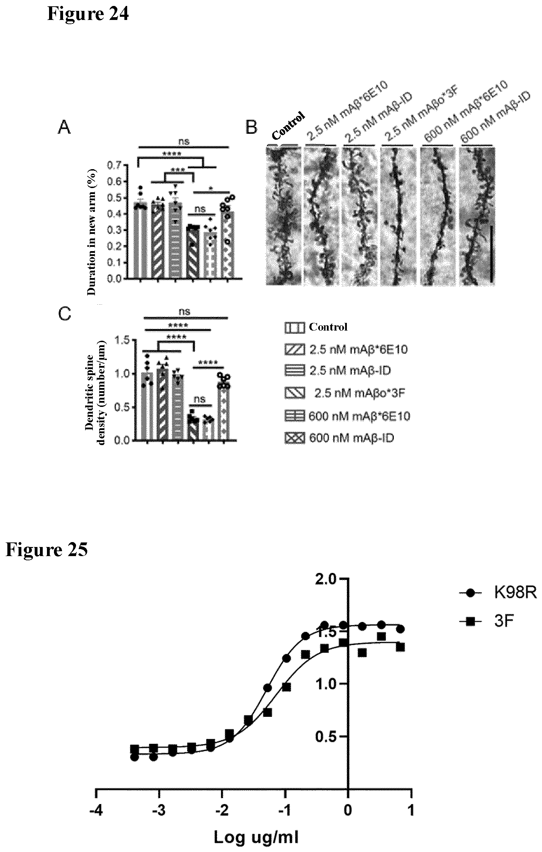

Figure 24 shows that Aβo*3F significantly reduces the cognitive function of mice and damages neurons in the mouse brain:
   A: The duration of each group of mice in the new arm in the Y-maze experiment;
   B: The density of dendritic spines in mouse neurons was detected through Golgi staining;
   C: Statistical analysis of dendritic spine density in item B above.
Figure 25 shows that after K at position 98 of the single-chain antibody heavy chain was mutated to R, the affinity was further improved, suggesting that the amino acid residue at position 98 plays an important role in the binding process of the single-chain antibody to Aβ oligomers.

### DETAILED DESCRIPTION

### Definitions

The term "amyloid proteins" as used herein include amyloid-β protein, microtubule-associated protein tau, α-synuclein, huntingtin protein, pancreatic amyloid protein, superoxide dismutase 1 (SOD1) and TDP-43 protein, including their monomers, oligomers, pre-fibrils or fibrils.

The term "toxic forms of amyloid proteins" as used herein refer to forms of amyloid proteins that play a negative role in the occurrence and development of neurodegenerative diseases, such as oligomeric forms, pre-fibrillar forms, etc.

The term "amyloid protein oligomers" as used herein refer to non-fibrillar aggregates formed by the aggregation of two or more amyloid monomer molecules.

The term "antibody" as used herein refers to an immunoglobulin molecule or a fragment of an immunoglobulin molecule which has the ability to bind to an epitope of an antigen. Naturally occurring antibodies typically comprise a tetramer which is usually composed of at least two heavy (H) chains and at least two light (L) chains. Immunoglobulins include the following isotypes: IgG, IgA, IgM, IgD, and IgE, and the corresponding heavy chains thereof are µ chain, δ chain, γ chain, α chain, and ε chain, respectively. The same class of Igs can be divided into different subclasses according to the differences in the amino acid compositions of the hinge regions and the numbers and positions of the heavy chain disulfide bonds, such as IgG can be divided into IgG1, IgG2, IgG3, IgG4 subtypes, and IgA can be divided into IgA₁ and IgA₂ subtypes. Light chains can be divided into κ chains and λ chains according to the differences in constant regions. The antibody of the invention may be of any isotype. The choice of isotype typically will be guided by the desired effector functions, such as ADCC induction. Exemplary isotypes are IgG1, IgG2, IgG3, and IgG4. Either of the human light chain constant domains, κ or λ, may be used. If desired, the class of the antibody of the present invention may be switched by known methods. For example, an antibody of the present invention that was originally IgG may be class switched to an IgM antibody of the present invention. Further, class switching techniques may be used to convert one IgG subclass to another, for instance from IgGl to IgG2. Thus, the effector function of the antibodies of the present invention may be changed by isotype switching to, e.g., an IgG1, IgG2, IgG3, IgG4, IgD, IgA, IgE, or IgM antibody for various therapeutic uses, provided that the complement (such as C1q and/or Fc receptor) binding activity of the antibody is reduced or eliminated. In some embodiments, the antibody of the present invention is an IgM or an antibody of IgG1, 2, 3 or 4 type. An antibody is said to be of a particular isotype if its amino acid sequence is most homologous to that isotype, relative to other isotypes.

Herein, the term "antibody" is used in the broadest sense thereof, and refers to a protein comprising an antigen-binding site, encompassing natural and artificial antibodies of various structures, including but not limited to intact antibodies and antigen-binding fragments of antibodies.

A "variable region" or "variable domain" is a domain of a heavy or light chain of an antibody involving in the binding of the antibody to its antigen. Each heavy chain of an antibody consists of a heavy chain variable region (herein referred to as VH) and a heavy chain constant region (herein referred to as CH), wherein the heavy chain constant region usually consists of 3 domains (CH1, CH2 and CH3). Each light chain consists of a light chain variable region (herein referred to as VL) and a light chain constant region (herein referred to as CL). The variable regions of the heavy and light chains are typically responsible for antigen recognition, while the constant domains of the heavy and light chains can mediate the binding of the immunoglobulin with host tissues or factors (including various cells of the immune system (e.g., effector cells), Fc receptors and the first component of the classical complement system (C1q)). The heavy and light chain variable regions comprise binding regions interacting with an antigen. The VH and VL regions can be further subdivided into hypervariable regions (HVRs) called "complementarity determining regions (CDRs)", which are interspersed with more conserved regions called "framework regions (FRs)". Each VH or VL consists of three CDR domains and four FR domains, arranged from the amino-terminus to the carboxy-terminus in the following order: FR1 - CDR1 - FR2 - CDR2 - FR3 - CDR3 - FR4.

The terms "complementarity determining region" or "CDR region" or "CDR" (used interchangeably herein with the hypervariable region "HVR") refer to a region in the antibody variable domains which is hypervariable in sequence and forms a loop structurally determined ("a hypervariable loop") and/or comprises antigen-contacting residues ("antigen-contacting points"). The CDRs are primarily responsible for binding to an epitope. Herein, the three CDRs of the heavy chain are referred to as HCDR1, HCDR2 and HCDR3, and the three CDRs of the light chain are referred to as LCDR1, LCDR2 and LCDR3.

It should be noted that the boundaries of the CDRs of the variable regions of the same antibody obtained based on different numbering systems may be different from each other. That is, the CDR sequences of the variable regions of the same antibody defined under different numbering systems are somewhat different from each other. Thus, when defining an antibody with the particular CDR sequences as defined in the invention, the scope of said antibody also covers antibodies of which the variable region sequences comprise said particular CDR sequences, but due to the different numbering system(s) used (such as different numbering system rules or combinations thereof), the CDR boundaries claimed by them might be different from the specific CDR boundaries defined in the invention.

The terms "mAbs", "monoclonal antibodies" or "monoclonal antibody composition" as used herein refer to a preparation of antibody molecules of single molecular composition, and refer to antibodies obtained from a substantially homogeneous population of antibodies, i.e., the population comprising individual antibodies are identical except for the naturally occurring mutations that may be present in minor amounts. A conventional monoclonal antibody composition displays a single binding specificity and affinity for a particular epitope. In certain embodiments, a monoclonal antibody can be composed of more than one Fab domain thereby increasing the specificity to more than one target. The term "monoclonal antibody" or "monoclonal antibody composition" is not limited by any particular method of production (e.g., recombinant, transgenic, hybridoma, etc.).

The present invention also includes "bispecific antibodies", wherein the antibodies of the present invention are part of a bivalent or multivalent bispecific framework targeting more than one epitope (for example, the second epitope may include an epitope of an active transport receptor, so that the bispecific antibody will exhibit improved transcytosis across biological barriers (such as the blood-brain barrier)). Therefore, in other embodiments, the monovalent Fab of the antibody of the present invention can be linked to another Fab or scFv targeting a different protein to produce a bispecific antibody. Bispecific antibodies can have dual functions, such as the therapeutic function conferred by the present invention and the transport function that can bind to receptor molecules to enhance transfer across biological barriers (such as the blood-brain barrier). The terms "double antibody", "bifunctional antibody", "bispecific antibody" or "BsAb" refers to an antibody which has two different antigen binding sites, so that which can simultaneously bind to two target antigens, alternatively which plays a role in mediating another special function simultaneously besides the targeting effect of the antibody. The special function effector molecules mediated can also be toxins, enzymes, cytokines, radionuclides, etc. The two arms of the bispecific antibody binding to an antigen can be from Fab, Fv, ScFv or dSFv, etc.

The term "antigen-binding fragment of antibody" refers to a fragment, a part, a region, or a domain of an antibody capable of binding to an epitope (e.g., obtainable by cleavage, recombination, synthesis, etc.). An antigen-binding fragment may comprise 1, 2, 3, 4, 5 or all 6 CDR domains of such antibodies and, while capable of binding to the epitope, it may exhibit different specificities, affinities or selectivities. Preferably, an antigen-binding fragment comprises all 6 CDR domains of said antibody. An antigen-binding fragment of an antibody may be a part of a single polypeptide chain (e.g., scFv) or comprise a single polypeptide chain, or may be a part of two or more polypeptide chains (each having an amino- and carboxy-terminus) (e.g., a bispecific antibody, a Fab fragment, a F(ab')₂ fragment, etc.) or comprise two or more polypeptide chains.

Examples of an antigen-binding fragment of the invention include (a) a Fab' or Fab fragment, a monovalent fragment consisting of the VL, VH, CL and CH1 domains; (b) F(ab')₂ fragments, bivalent fragments comprising two Fab fragments linked by a disulfide bond at the hinge domain; (c) an Fd fragment consisting of the VH and CH1 domains; (d) a Fv fragment consisting of VL and VH domains of a single arm of an antibody; (e) a single chain Fv (scFv), a recombinant protein formed by linking VH and VL domains of an antibody with a peptide linker by genetic engineering; (f) a dAb fragment (Ward et al., Nature, 341, 544 -546 (1989)), which consists essentially of a VH domain and also called domain antibodies (Holt et al., Trends Biotechnol., 2i(11): 484-90); (g) camelid or nanobodies (Revets et al., Expert Opin Biol Ther., 5(1): 111-24) and (h) an isolated complementarity determining region (CDR).

In some embodiments, the antibodies and antigen-binding fragments thereof of the present invention are single-chain antibodies. In some embodiments, the present invention provides single-chain Fv (scFv), wherein the heavy chain and light chain in the Fv of the antibody of the present invention are connected into a single peptide chain by a flexible peptide linker (typically about 10, 12, 15 or more amino acid residues). Methods for producing such antibodies are described in, for example, US 4,946,778; Pluckthun, The Pharmacology of Monoclonal Antibodies, Vol. 113, Rosenburg and Moore ed., Springer-Verlag, New York, pp. 269-315 (1994); Bird et al., Science, 242, 423-426 (1988); Huston et al., PNAS USA 85, 5879-5883 (1988) and McCafferty et al., Nature, 348, 552-554 (1990). Single-chain antibodies are monovalent if only a single VH and VL are used, bivalent if two VHs and VLs are used, or multivalent if more than two VHs and VLs are used.

In some embodiments, the antibodies and antigen-binding fragments thereof of the present invention are chimeric antibodies. The term "chimeric antibody" means that a portion of the heavy and/or light chains is identical or homologous to the corresponding sequences of an antibody from a particular species or belonging to a particular antibody class or subclass, while the rest of the chains is identical or homologous to the corresponding sequences of an antibody from another species or belonging to another antibody class or subclass, providing that they exhibit the desired biological activity. The invention provides the variable region antigen binding sequences from human antibodies. Thus, chimeric antibodies primarily focused herein include antibodies which comprise one or more human antigen-binding sequences (e.g., CDRs) and comprise one or more sequences from non-human antibodies, such as FR or C region sequences. Furthermore, chimeric antibodies described herein are antibodies comprising human variable region antigen-binding sequence from one antibody class or subclass and other sequences from another antibody class or subclass, such as FR or C region sequences.

In some embodiments, the antibodies and antigen-binding fragments thereof of the present invention are humanized antibodies. The term "humanized antibody" refers to an antibody in which CDR sequences from the germline of another mammalian species, such as a mouse, have been grafted among the human framework region sequences. Additional framework region modifications can be made within the human framework region sequences.

In some embodiments, the antibodies and antigen-binding fragments thereof of the invention are human antibodies or fully human antibodies. The terms "human antibody" or "fully human antibody" ("humAb" or "HuMab") refers to an antibody comprising variable and constant domains derived from human germline immunoglobulin sequences. The human antibodies of the invention may include amino acid residues not encoded by human germline immunoglobulin sequences (e.g., mutations introduced by random or site-specific mutagenesis *in vitro* or during gene rearrangement or by somatic mutation *in vivo*).

Variant antibodies are also included within the scope of the invention. Accordingly, variants of the sequences recited in the invention are also included within the scope of the invention. Other variants of antibody sequences with improved affinity can be obtained by using methods known in the art and these variants are also included within the scope of the invention. For example, amino acid substitutions can be used to obtain antibodies with further improved affinity. Alternatively, codon optimization of nucleotide sequences can be used to improve the translation efficiency of expression systems in antibody production.

Such variant antibody sequences have 70% or more (e.g., 80%, 85%, 90%, 95%, 97%, 98%, 99% or more) sequence homology to the sequences recited in the invention. Such a sequence homology is obtained by calculation relative to the full length of the reference sequence (i.e., the sequence recited in the invention).

The numbering of amino acid residues in the invention is according to IMGT^{®}, the international ImMunoGeneTics information system^{®} or Kabat, E. A., Wu, T. T., Perry, H. M., Gottesmann, K. S. & Foeller, C. (1991). Sequences of Proteins of Immunological Interest, 5th edit., NIH Publication no. 91-3242 U.S. Department of Health and Human Services; Chothia, C. & Lesk, A. M. (1987). Canonical Structures For The Hypervariable domains Of Immunoglobulins. J. Mol. Biol. 196, 901-917. Unless otherwise specified, the numbering of amino acid residues in the invention is according to the Kabat numbering system.

An antibody or an antigen-binding fragment thereof is said to "specifically" bind a region of another molecule (*i.e.,* an epitope) if it reacts or associates more frequently, more rapidly, with greater duration and/or with greater affinity or avidity with that epitope relative to alternative epitopes. In some embodiments, the antibody or antigen-binding fragment thereof of the invention binds to a human amyloid protein, particularly its toxic form, with an affinity of at least 10⁻⁷M, such as 10⁻⁸ M, 10⁻⁹ m, 10⁻¹⁰ M, 10⁻¹¹ M or higher. Preferably, the antibody or antigen-binding fragment thereof binds under physiological conditions (e.g., *in vivo*). Therefore, specifically binding to an amyloid protein, particularly its toxic form, refers to the ability of the antibody or antigen-binding fragment thereof to bind to an amyloid protein, particularly its toxic form, with the above-mentioned specificity and/or under such conditions. Methods suitable for determining such a binding are known in the art.

The term "binding" in the context of the binding of an antibody to a predetermined antigen typically refers to binding with an affinity corresponding to a KD of about 10⁻⁶ M or less, which is at least ten-fold lower, such as at least 100-fold lower, for instance at least 1,000-fold lower than its affinity for binding to a non-specific antigen (e.g., BSA, casein) other than the predetermined antigen or a closely-related antigen.

As used herein, the term "kd" (sec -1 or 1/s) refers to the dissociation constant of a particular antibody-antigen interaction. Said value is also referred as the k_{off} value.

As used herein, the term "ka" (M-1 x sec-1 or 1/Msec) refers to the association rate constant of a particular antibody-antigen interaction.

As used herein, the term "KD" (M) refers to the dissociation equilibrium constant of a particular antibody-antigen interaction and is obtained by dividing the kd by the ka.

As used herein, the term "KA" (M-1 or 1/M) refers to the association equilibrium constant of a particular antibody-antigen interaction and is obtained by dividing the ka by the kd.

The fact that a single amino acid alteration of a CDR residue can result in loss of functional binding (Rudikoff, S. et al. (1982), "Single Amino Acid Substitution Altering Antigen-Binding Specificity", Proc. Natl. Acad. Sci. (USA) 79(6):1979-1983) provides a means for systematically identifying alternative functional CDR sequences. In one preferred method for obtaining such variant CDRs, a polynucleotide encoding the CDR is mutagenized (for example via random mutagenesis or by a site-directed method (e.g., polymerase chain-mediated amplification with primers that encode the mutated locus)) to produce a CDR having a substituted amino acid residue. By comparing the identity of the relevant residue in the original (functional) CDR sequence to the identity of the substituted (non-functional) variant CDR sequence, the BLOSUM62.iij substitution score for that substitution can be identified. The BLOSUM system provides a matrix of amino acid substitutions created by analyzing a database of sequences for trusted alignments (Eddy, S.R. (2004), "Where Did The BLOSUM62 Alignment Score Matrix Come From?", Nature Biotech. 22(8):1035-1036; Henikoff, J.G. (1992), "Amino acid substitution matrices from protein blocks", Proc. Natl. Acad. Sci. (USA) 89: 10915-10919; Karlin, S. et al. (1990), "Methods For Assessing The Statistical Significance Of Molecular Sequence Features By Using General Scoring Schemes", Proc. Natl. Acad. Sci. (USA) 87:2264-2268; Altschul, S.F. (1991), "Amino Acid Substitution Matrices From An Information Theoretic Perspective", J. Mol. Biol. 219, 555-565. Currently, the most advanced BLOSUM database is the BLOSUM62 database (BLOSUM62.iij). **Table 1** presents the BLOSUM62.iij substitution scores (the higher the score the more conservative the substitution and thus the more likely the substitution will not affect function). If an antigen-binding fragment comprising the resultant CDR fails to bind to an amyloid protein, for example, then the BLOSUM62.iij substitution score is deemed to be insufficiently conservative, and a new candidate substitution is selected and produced having a higher substitution score. Thus, for example, if the original residue was glutamate (E), and the non-functional substitute residue was histidine (H), then the BLOSUM62.iij substitution score will be 0, and more conservative changes (such as to aspartate, asparagine, glutamine, or lysine) are preferred.

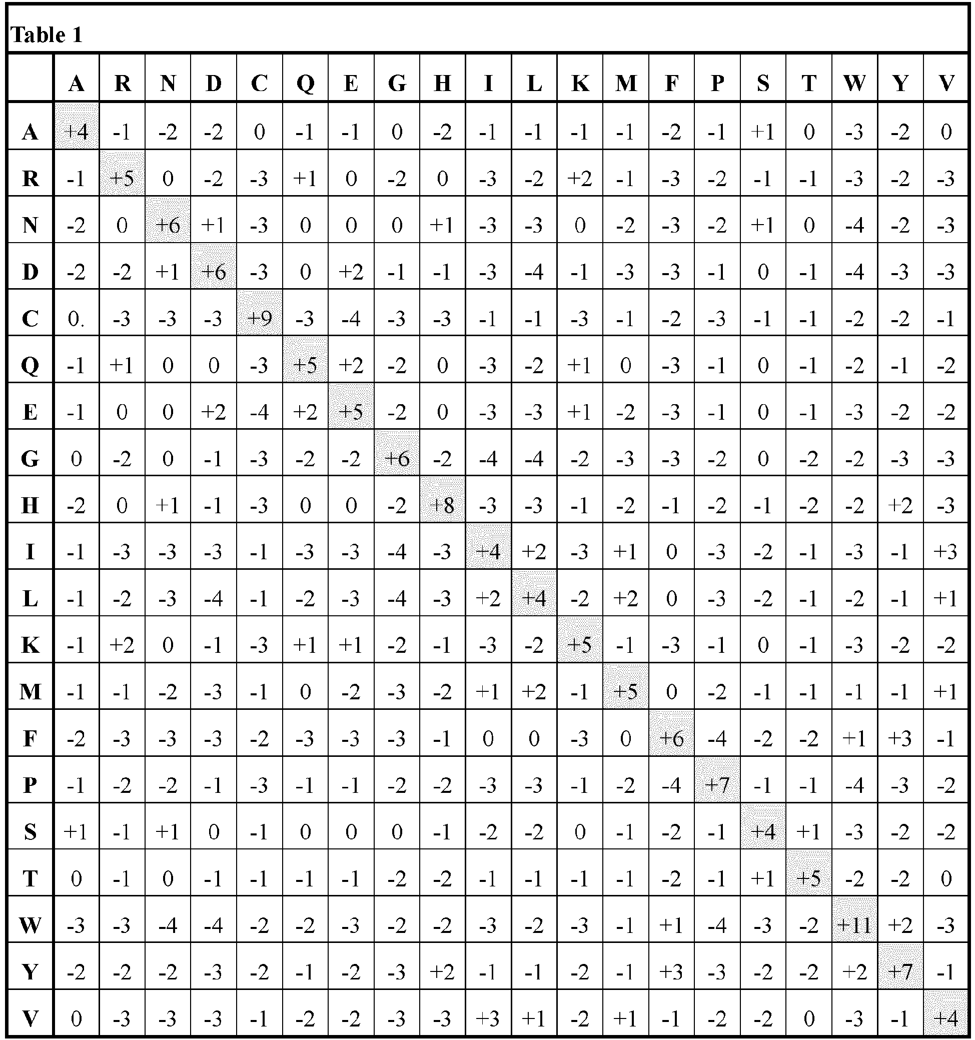

The invention thus contemplates the use of random mutagenesis to identify improved CDRs. In the context of the present invention, conservative substitutions may be defined by substitutions within the classes of amino acids reflected in one or more of Tables 2, 3, or 4:

### Amino Acid Residue Classes For Conservative Substitutions:

| | **Table 2** | |
|---|---|---|
| Acidic residue | | Asp (D) and Glu (E) |
| Basic residues | | Lys (K), Arg (R), and His (H) |
| Hydrophilic uncharged residues | | Ser (S), Thr (T), Asn (N) and Gln (Q) |
| Aliphatic uncharged residue | | Cly (G), Ala (A), Val (V), Leu (L) and Ile (I) |
| Nonpolar uncharged residues | | Cys (C), Met (M), and Pro (P) |
| Aromatic residues | | Phe (F), Tyr (Y) and Trp (W) |

### Alternative Conservative Amino Acid Residue Substitution Classes:

| **Table 3** | | | |
|---|---|---|---|
| **1** | **A** | **S** | **T** |
| **2** | **D** | **E** | |
| **3** | **N** | **Q** | |
| **4** | **R** | **K** | |
| **5** | **I** | **L** | **M** |
| **6** | **F** | **Y** | **W** |

### Alternative Physical and Functional Classifications of Amino Acid Residues:

| | **Table 4** | |
|---|---|---|
| Alcohol Group-Containing Residues | | S and T |
| Aliphatic Residues | | I, L, V and M |
| Cycloalkenyl-Associated Residues | | F, H, W and Y |
| Hydrophobic Residues | | A, C, F, G, H, I, L, M, R, T, V, W and Y |
| Negatively Charged Residues | | D and E |
| Polar Residues | | C, D, E, H, K, N, Q, R, S and T |
| Positively Charged Residues | | H, K and R |
| Small Residues | | A, C, D, G, N, P, S, T and V |
| Very Small Residues | | A, G and S |
| Residues Involved In Turn Formation | | A, C, D, E, G, H, K, N, Q, R, S, P and T |
| Flexible Residues | | Q, T, K, S, G, P, D, E and R |

More conservative substitutions groupings include: valine-leucine-isoleucine, phenylalanine-tyrosine, lysine-arginine, alanine-valine, and asparagine-glutamine.

In some embodiments, the hydrophilic amino acid is selected from the group consisting of Arg, Asn, Asp, Gln, Glu, His, Tyr, and Lys.

Additional groups of amino acids may also be formulated using the principles described in, e.g., Creighton (1984) Proteins: Structure and Molecular Properties (2d Ed. 1993), W. H. Freeman and Company.

Thus, the sequences of the CDR variants comprised in antibodies or antigen-binding fragments thereof may differ from those of the CDRs of the parent antibody by substitutions, such as substitutions of 4, 3, 2 or 1 amino acid residues. According to an embodiment of the invention, amino acid(s) in the CDR regions may be substituted with conservative substitutions, as defined in 3 Tables above.

"Homology" or "sequence identity" refers to the percentage of residues in a variant polynucleotide or polypeptide sequence that are identical to those of the non-variant sequence, after alignment of the sequences and introduction of gaps. In specific embodiments, polynucleotide and polypeptide variants have at least about 70%, at least about 75%, at least about 80%, at least about 90%, at least about 95%, at least about 98%, or at least about 99% polynucleotide or polypeptide homology.

Such variant polypeptide sequences have 70% or higher (i.e., 80%, 85%, 90%, 95%, 97%, 98%, 99% or higher) sequence identity to the sequences recited in the invention. In other embodiments, the invention provides polypeptide fragments comprising contiguous stretches of various lengths of the amino acid sequences disclosed herein. For example, where applicable, the peptide sequences provided herein comprise at least about 5, 10, 15, 20, 30, 40, 50, 75, 100, 150 or more, and all intermediate lengths therebetween, contiguous amino acid residues of one or more sequences disclosed herein.

Antibodies of the invention may be monoclonal antibodies produced by recombinant DNA techniques.

In some embodiments, the antibody of the present invention is a full-length antibody, preferably an IgG1-4 antibody or an IgM antibody. In other embodiments, the antibody of the present invention is an antigen-binding fragment of an antibody or a single-chain antibody.

The subunit structures and three-dimensional configuration of the constant regions of the various immunoglobulin classes are well known. The term "VH domain" as used herein includes the amino terminal variable domain of an immunoglobulin heavy chain and the term "CH1 domain" includes the first (most amino terminal) constant region domain of an immunoglobulin heavy chain. The CH1 domain is adjacent to the VH domain and is amino terminal to the hinge region of an immunoglobulin heavy chain molecule. As used herein, the term "CH2 domain" includes the portion of a heavy chain molecule that extends, e.g., from about residue 244 to residue 360 of an antibody using conventional numbering schemes (residues 244 to 360, Kabat numbering system; and residues 231-340, EU numbering system; see Kabat et al., U.S. Dept, of Health and Human Services, "Sequences of Proteins of Immunological Interest" (1983). The CH2 domain is unique in that it is not closely paired with another domain. Rather, two N-linked branched carbohydrate chains are interposed between the two CH2 domains of an intact native IgG molecule. It is also well documented that the CH3 domain extends from the CH2 domain to the C-terminal of the IgG molecule and comprises approximately 108 residues. As used herein, the term "hinge region" includes the portion of a heavy chain molecule that joins the CH1 domain to the CH2 domain. This hinge region comprises approximately 25 residues and is flexible, thus allowing the two N-terminal antigen binding regions to move independently. Hinge regions can be subdivided into three distinct domains: upper, middle, and lower hinge domains (Roux et al, J. Immunol 161: 4083 (1998)).

In some embodiments, the antibody of the present invention is a monovalent antibody, preferably a monovalent antibody as described in WO2007059782 (which is incorporated herein by reference in its entirety) having a deletion of the hinge region. Accordingly, in some embodiments, the antibody is a monovalent antibody, wherein said antibody is constructed by a method comprising: i) providing a nucleic acid construct encoding the light chain of said monovalent antibody, said construct comprising a nucleotide sequence encoding the VL region of a selected antigen-specific antibody and a nucleotide sequence encoding the constant CL region of an Ig, wherein said nucleotide sequence encoding the VL region of a selected antigen specific antibody and said nucleotide sequence encoding the CL region of an Ig are operably linked together, and wherein, in case of an IgG1 subtype, the nucleotide sequence encoding the CL region has been modified such that the CL region does not contain any amino acids capable of forming disulfide bonds or covalent bonds with other peptides comprising an identical amino acid sequence of the CL region in the presence of polyclonal human IgG or when administered to an animal or human being; ii) providing a nucleic acid construct encoding the heavy chain of said monovalent antibody, said construct comprising a nucleotide sequence encoding the VH region of a selected antigen specific antibody and a nucleotide sequence encoding a constant CH region of a human Ig, wherein the nucleotide sequence encoding the CH region has been modified such that the region corresponding to the hinge region and, as required by the Ig subtype, other regions of the CH region, such as the CH3 region, does not comprise any amino acid residues which participate in the formation of disulphide bonds or covalent or stable non-covalent inter-heavy chain bonds with other peptides comprising an identical amino acid sequence of the CH region of the human Ig in the presence of polyclonal human IgG or when administered to an animal human being, wherein said nucleotide sequence encoding the VH region of a selected antigen specific antibody and said nucleotide sequence encoding the CH region of said Ig are operably linked together; iii) providing a cell expression system for producing said monovalent antibody; iv) producing said monovalent antibody by co-expressing the nucleic acid constructs of (i) and (ii) in cells of the cell expression system of (iii).

Similarly, in some embodiments, the antibody of the invention is a monovalent antibody, which comprises:
(i) a variable domain of an antibody of the invention as described herein or an antigen-binding part of the said domain, and
(ii) a CH domain of an immunoglobulin or a domain thereof comprising the CH2 and CH3 domains, wherein the CH domain or domain thereof has been modified such that the domain corresponding to the hinge domain and, if the immunoglobulin is not an IgG4 subtype, other domains of the CH domain, such as the CH3 domain, do not comprise any amino acid residues, which are capable of forming disulfide bonds with an identical CH domain or other covalent or stable non-covalent inter-heavy chain bonds with an identical CH domain in the presence of polyclonal human IgG.

In some other embodiments, the heavy chain of the monovalent antibody of the invention has been modified such that the entire hinge region has been deleted.

In other embodiments, the sequence of the monovalent antibody has been modified so that it does not comprise any acceptor sites for N-linked glycosylation.

Antibodies of the invention are produced by any technique known in the art, such as, but not limited to, any chemical, biological, genetic, or enzymatic technique, alone or in combination. In general, if knowing the amino acid sequence of a desired sequence, one skilled in the art can readily generate such an antibody by standard techniques used to generate polypeptides. For example, these antibodies can be synthesized using well-known solid-phase methods, preferably using a commercially available peptide synthesis apparatus (such as that manufactured by Applied Biosystems, Foster City, California) and following the manufacturer's instructions. Alternatively, antibodies of the invention can be synthesized by recombinant DNA techniques well known in the art. For example, after incorporating a DNA sequence encoding an antibody into an expression vector and introducing these vectors into eukaryotic or prokaryotic host cells suitable for expressing the desired antibodies, antibodies as DNA expression products can be obtained, which can then be isolated from the host cells using known techniques.

The antibodies and antigen-binding fragments thereof described herein may be modified by inclusion of any suitable number of modified amino acids and/or associations with such conjugated substituents. Suitability in this context is generally determined by the ability to retain the amyloid protein, particularly its toxic form, selectivity and/or the amyloid protein, particularly its toxic form, specificity at least substantially associated with the non-derivatized parent antibody. The inclusion of one or more modified amino acids may be advantageous in, for example, increasing polypeptide serum half-life, reducing polypeptide antigenicity, or increasing polypeptide storage stability. Amino acid(s) are modified, for example, co-translationally or post-translationally during recombinant production (e.g., N-linked glycosylation at N-X-S/T motifs during expression in mammalian cells) or modified by synthetic means. Non-limiting examples of a modified amino acid include a glycosylated amino acid, a sulfated amino acid, a prenylated (e.g., farnesylated, geranyl-geranylated) amino acid, an acetylated amino acid, an acylated amino acid, a PEGylated amino acid, a biotinylated amino acid, a carboxylated amino acid, a phosphorylated amino acid, and the like. References adequate to guide one of skill in the modification of amino acids are replete throughout the literature, see e.g., Walker (1998) Protein Protocols On CD-Rom, Humana Press, Totowa, NJ. The modified amino acid may, for instance, be selected from a glycosylated amino acid, a PEGylated amino acid, a farnesylated amino acid, an acetylated amino acid, a biotinylated amino acid, an amino acid conjugated to a lipid moiety, or an amino acid conjugated to an organic derivatizing agent.

The antibodies and antigen-binding fragments thereof of the invention may also be chemically modified by covalent conjugation to a polymer to for instance increase their circulating half-life. Exemplary polymers and methods to attach them to peptides are illustrated in for instance US 4,766,106; US 4,179,337; US 4,495,285 and US 4,609,546. Additional illustrative polymers include polyoxyethylated polyols and polyethylene glycol (PEG) (e.g., a PEG with a molecular weight of between about 1,000 and about 40,000 D, such as between about 2,000 and about 20,000 D, e.g., about 3,000-12,000 D).

The antibodies and antigen-binding fragments thereof of the present invention also include mutants formed by substitutions, deletions, or additions of one or more amino acid residues of the above-mentioned amyloid protein antibodies.

In a further aspect, the present invention relates to an expression vector comprising nucleotide sequences encoding one or more polypeptide chains of the antibody or antigen-binding fragment thereof of the invention. Such expression vectors can be used to recombinantly produce the antibodies and antigen-binding fragments thereof of the invention.

An expression vector in the context of the present invention may be any suitable DNA or RNA vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In some embodiments, an antibody-encoding nucleic acid is comprised in a naked DNA or RNA vector, including, for example, a linear expression element (as described in, for instance, Sykes and Johnston, Nat Biotech 12, 355-59 (1997)), a compacted nucleic acid vector (as described in for instance US 6,077,835 and/or WO 00/70087), a plasmid vector such as pBR322, pUC19/18, or pUC118/119, a minimally-sized nucleic acid vector (as described in, for instance, Schakowski et al., MoI Ther 3, 793-800 (2001)), or as a precipitated nucleic acid vector construct, such as a CaPO4-precipitated construct (as described in, for instance, WO 00/46147, Benvenisty and Reshef, PNAS USA 83, 9551-55 (1986), Wigler et al., Cell 14, 725 (1978), and Coraro and Pearson, Somatic Cell Genetics 2, 603 (1981)). Such nucleic acid vectors and the usage thereof are well known in the art (see for instance US 5,589,466 and US 5,973,972).

In some embodiments, the vector is suitable for expressing the antibody or antigen-binding fragment thereof of the present invention in bacterial cells. Examples of such vectors include, for example, BlueScript (Stratagene), pIN vector (Van Heeke & Schuster, J Biol Chem, 264, 5503-5509 (1989)), pET vector (Novagen, Madison, Wisconsin), etc.

An expression vector may also be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in: F. Ausubel et al., ed. Current Protocols in Molecular Biology, Greene Publishing and Wiley InterScience New York (1987), Grant et al., Methods in Enzymol 153, 516-544 (1987), Mattanovich, D. et al. Methods Mol. Biol. 824, 329-358 (2012), Celik, E. et al. Biotechnol. Adv. 30(5), 1108-1118 (2012), Li, P. et al. Appl. Biochem. Biotechnol. 142(2), 105-124 (2007), Böer, E. et al. Appl. Microbiol. Biotechnol. 77(3), 513-523 (2007), van der Vaart, J.M. Methods Mol. Biol. 178, 359-366 (2002), and Holliger, P. Methods Mol. Biol. 178, 349-357 (2002)).

In an expression vector of the invention, the antibody-encoding nucleic acids may comprise or be associated with any suitable promoter, enhancer, and other expression-facilitating elements. Examples of such elements include strong expression promoters (e.g., human CMV IE promoter/enhancer as well as RSV, SV40, SL3-3, MMTV, and HIV LTR promoters), effective poly (A) termination sequences, an origin of replication for plasmid product in E. coli, an antibiotic resistance gene as selectable marker, and/or a convenient cloning site (e.g., a polylinker). Nucleic acids may also comprise an inducible promoter as opposed to a constitutive promoter such as CMV IE (the skilled artisan will recognize that such terms are actual descriptors of a degree of gene expression under certain conditions).

In an even further aspect, the invention relates to a recombinant eukaryotic or prokaryotic host cell, such as a transfectoma, which produces an antibody or epitope-binding fragment thereof of the invention as defined herein or a bispecific molecule of the invention as defined herein. Examples of host cells include yeast, bacteria, and mammalian cells, such as CHO or HEK cells. For example, in some embodiments, the present invention provides a cell comprising a nucleic acid stably integrated into the cellular genome that comprises a sequence coding for expression of an antibody of the present invention or an antigen-binding fragment thereof. In other embodiments, the present invention provides a cell comprising a non-integrated nucleic acid, such as a plasmid, cosmid, phagemid, or linear expression element, which comprises a sequence coding for expression of an antibody or antigen-binding fragment thereof of the invention.

Antibodies and antigen-binding fragments thereof of the invention can be produced in different cell lines, such as human cell lines, non-human mammalian cell lines, and insect cell lines, such as CHO cell lines, HEK cell lines, BHK-21 cell lines, murine cell lines (such as myeloma cell line), fibrosarcoma cell line, PER.C6 cell line, HKB-11 cell line, CAP cell line, and HuH-7 human cell line (Dumont et al., 2015, Crit Rev Biotechnol., Sep. 18, 1-13., the contents of which are incorporated herein by reference).

The antibodies of the invention are suitably separated from the culture medium by conventional immunoglobulin purification methods, such as Protein A-Sepharose, hydroxyapatite chromatography, gel electrophoresis, dialysis, or affinity chromatography.

The present invention further relates to a composition comprising, consisting of, or consisting essentially of the antibodies of the invention.

As used herein, the term "consisting essentially of" with respect to a composition means that at least one antibody of the invention as described above is the only biologically active therapeutic agent or reagent in the composition.

In one embodiment, the composition of the present invention is a pharmaceutical composition and further comprises a pharmaceutically acceptable excipient, diluent, or carrier.

The present invention further relates to a medicament comprising, consisting of, or consisting essentially of an antibody of the present invention and further comprising a pharmaceutically acceptable excipient, diluent, or carrier.

The term "pharmaceutically acceptable carrier" refers to an excipient that does not produce adverse, allergic, or other adverse reactions when administered to animals, preferably humans, including any, and all solvents, dispersion media, coatings, antibacterial and antifungal agents, isotonic and absorption delaying agents and the like. For human administration, preparations should meet sterility, pyrogenicity, general safety and purity standards as required by regulatory agencies (e.g., FDA or EMA).

In some embodiments, the glycosylation of the antibodies of the invention is modified. For example, an aglycoslated antibody can be made (i.e., the antibody lacks glycosylation). Glycosylation can be altered to, for example, increase the affinity of the antibody for an antigen or change the ADCC activity of the antibody. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for an antigen. Such an approach is described in further detail in U.S. Patent Nos. 5,714,350 and 6,350,861 by Co et al. Additionally, or alternatively, an antibody can be made that has an altered type of glycosylation. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation.

Recombinant expression vectors can be introduced into host cells to produce a transformed host cell. The terms "transformed with", "transfected with", "transformation" and "transfection" are intended to encompass introduction of nucleic acid (e.g., a vector) into a cell by one of many possible techniques known in the art. The terms "transformed host cell" or "transduced host cell" as used herein are intended to also include cells that have been transformed with a recombinant expression vector of the present invention. Prokaryotic cells can be transformed with nucleic acid by, for example, electroporation or calcium-chloride mediated transformation. For example, nucleic acid can be introduced into mammalian cells via conventional techniques such as calcium phosphate or calcium chloride co-precipitation, DEAE-dextran mediated transfection, lipofection, electroporation or microinjection. Suitable methods for transforming and transfecting host cells can be found in Sambrook *et al*., 1989, and other laboratory textbooks.

Although the nucleotide sequence defined above is DNA, in an alternative embodiment of the invention, the nucleotide sequence may be RNA. Thus, RNA sequences corresponding to the DNA sequences described herein are encompassed. The skilled person will understand how to derive the RNA sequences encoding the same protein/polypeptide products as the DNA sequences shown above. "T" should be replaced with "U".

As used herein, the terms "nucleic acid sequence" or "nucleic acid molecule" or "polynucleotide" or "nucleotide sequence" refer to a sequence of nucleotides or nucleotide monomers consisting of naturally occurring bases, sugars, and inter-sugar (backbone) linkages. The term also includes modified or substituted sequences containing non-naturally occurring monomers or portions thereof. The nucleic acid, polynucleotide or nucleotide sequence of the invention may be a deoxyribonucleic acid sequence (DNA) or a ribonucleic acid sequence (RNA) and may include naturally occurring bases, including adenine, guanine, cytosine, thymidine, and uracil. The sequence may also contain modified bases. Examples of such modified bases include aza and deazaadenine, guanine, cytosine, thymidine, and uracil; and xanthines and hypoxanthines. The nucleic acid, polynucleotide or nucleotide sequence may be double-stranded or single-stranded. The nucleic acid, polynucleotide or nucleotide sequence may be wholly or partially synthetic or recombinant.

Although the antibodies, nucleic acids, vectors, or cells of the present invention can effectively fight diseases when used alone, additional therapeutic agents can be used in combination with the antibodies, nucleic acids, vectors, or cells of the present invention to fight diseases. Therefore, in another embodiment of the present invention, at least one additional or further therapeutic agent (e.g., other neurodegenerative disease therapeutic drugs) can be administered to the subject. Therefore, the antibodies or antigen-binding fragments thereof, nucleic acids, vectors or cells of the present invention and additional therapeutic agents (e.g., other neurodegenerative disease therapeutic drugs) can be administered to the subject. Therefore, the composition or pharmaceutical composition of the present invention may comprise other active agents or therapeutic agents, as well as the antibodies or antigen-binding fragments thereof, nucleic acids, vectors and/or cells of the present invention. However, it should be understood that the antibodies or antigen-binding fragments thereof, nucleic acids, vectors or cells of the present invention and other therapeutic agents (e.g., other neurodegenerative disease therapeutic drugs) can be administered separately, for example, by separate administration routes. **In** addition, the antibodies or antigen-binding fragments thereof, nucleic acids, vectors or cells of the present invention and at least one other therapeutic agent (e.g., other neurodegenerative disease such drugs) can be administered sequentially or (substantially) simultaneously. They can be administered in the same pharmaceutical preparation or drug, or they can be formulated and administered separately. For sequential administration, the additional therapeutic agent can be administered at least 1 minute, 10 minutes, 1 hour, 6 hours, 12 hours, 1 day, 5 days, 10 days, 2 weeks, 4 weeks, or 6 weeks before or after administration of the antibodies or antigen-binding fragments thereof/nucleic acids/vectors/cells.

"Pharmaceutically acceptable" includes preparations that are sterile and pyrogen free. Suitable pharmaceutical carriers, diluents and excipients are well known in the pharmaceutical art. A carrier must be "acceptable" in the sense of being compatible with the drug and not deleterious to the recipient thereof. Typically, the carrier will be water or saline (alternatively called an infusion solution), which will be sterile and pyrogen-free; however, other acceptable carriers may be used.

The pharmaceutical composition of the present invention can be administered in a manner suitable for the disease to be treated (or prevented). The number and frequency of administration will be determined by factors such as the patient's conditions and the type and severity of the patient's disease, although the appropriate dosage can be determined by clinical trials.

The composition of the present invention can be administered in a single dose or multiple doses. In particular, the composition can be administered in a single dose.

The antibodies or antigen-binding fragments thereof, nucleic acids, vectors or compositions of the present invention can be administered in the form of a pharmaceutical preparation comprising an active ingredient by any parenteral route. Depending on the condition and the patient to be treated and the route of administration, the composition can be administered in different doses. In any case, the physician will determine the actual dosage that is most suitable for any individual patient, and it will vary with the age, weight, and response of the particular patient.

In human therapy, the antibody or antigen-binding fragment thereof, nucleic acid or composition of the present invention is usually mixed with a suitable pharmaceutical excipient, diluent or carrier selected according to the expected route of administration and standard pharmaceutical practice. For each of the embodiments, the antibody or antigen-binding fragment thereof, nucleic acid molecule or composition of the present invention can be administered by a variety of dosage forms. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal, preferably intravenous), infusions, and combinations thereof. Typically, the cells of the present invention can be administered in injection or infusion buffer. Exemplary preparations can be found in, for example, Remington's Pharmaceutical Sciences, 19th edition., Grennaro, A., ed., 1995, which is incorporated herein by reference.

The antibodies or antigen-binding fragments thereof, nucleic acids or compositions of the present invention can also be administered parenterally, such as intravenously, intraarterially, intraperitoneally, intrathecally, intracranially, topically, intramuscularly, buccally, subcutaneously, epidermally, epidurally, inhaled, intracardially, intraventricularly, intraocularly, intraspinally, nasally, sublingually, transdermally or transmucosally, or they can be administered by infusion techniques. They are preferably used in the form of sterile aqueous solutions, which may contain other substances, such as enough salts or glucose to make the solution isotonic with the blood. If necessary, the aqueous solution should be appropriately buffered (preferably pH 3 to 9). Preparing suitable parenteral preparations under aseptic conditions can be easily accomplished by standard pharmaceutical techniques well known to those skilled in the art.

The formulations may be presented in unit-dose or multi-dose containers, such as sealed ampoules, bags, and vials.

In some embodiments, the antibody or antigen-binding fragment thereof, nucleic acid or composition of the present invention is used to treat and/or prevent a neurodegenerative disease in a subject, comprising administering to a subject in need thereof a therapeutically or prophylactically effective amount of the antibody or antigen-binding fragment thereof, nucleic acid or composition. **In** some embodiments, the neurodegenerative disease is selected from the group consisting of Alzheimer's disease, Parkinson's disease, Huntington's disease, frontotemporal dementia, amyotrophic lateral sclerosis, and spinocerebellar ataxia.

The terms "treatment" or "treating" as used herein mean ameliorating, slowing, attenuating, or reversing the progress or severity of a disease or disorder, or ameliorating, slowing, attenuating, or reversing one or more symptoms or side effects of such disease or disorder. **In** the present invention, the terms "treatment" or "treating" further mean an approach for obtaining beneficial or desired clinical results, wherein "beneficial or desired clinical results" include, without limitation, alleviation of a symptom, diminishment of the extent of a disorder or disease, stabilized (i.e., not worsening) disease or disorder state, delay or slowing of the progression a disease or disorder state, amelioration or palliation of a disease or disorder state, and remission of a disease or disorder, whether partial or total, detectable or undetectable.

The terms "prevention" or "preventing" refer to the administration of antibodies and functional fragments thereof of the present invention, thereby preventing or retarding the development of at least one symptom of a disease or condition. The term also includes treating a subject in remission to prevent or hinder relapse.

The term "subject" refers to a warm-blooded animal, preferably a mammal (including humans, domestic and farm animals, zoo animals, sport animals, or pet animals such as dogs, cats, cows, horses, sheep, pigs, goats, rabbits, etc.), more preferably humans. In one embodiment, a subject may be a "patient", i.e., a warm-blooded animal, more preferably a human, who is waiting to receive or is receiving medical care or will be a subject of a medical procedure, or monitoring for the development of a disease. In one embodiment, the subject is an adult (e.g., a subject over 18 years old). In another embodiment, the subject is a child (e.g., a subject under 18 years old). In one embodiment, the subject is a male. In another embodiment, the subject is a female.

The antibodies and antigen-binding fragments thereof of the present invention can be further used in diagnostic methods or as diagnostic imaging ligands. In some embodiments, the antibodies and antigen-binding fragments thereof of the present invention can be labeled or modified with radioactive labels, fluorescent labels, fluorescein-type labels, rhodamine-type labels, phycoerythrin, umbelliferone, lissamine, cyanine, Texas Red, BODIPY FL-SE^{®} (Invitrogen) or its analogs, horseradish peroxidase, alkaline phosphatase, β-galactosidase, acetylcholinesterase, streptavidin/biotin and avidin/biotin. Such labeled or modified antibodies or antigen-binding fragments thereof can be used to detect the presence and/or concentration of amyloid protein in a sample, respectively, for clinical diagnosis, experimental detection, etc. The experimental techniques used include, but are not limited to, ELISA, Dot-blot, Western-blot, chemiluminescence, electrochemiluminescence, Simoa technology, radioactive technology, etc. In some embodiments, paramagnetic labels can also be employed, and are preferably detected using Positron Emission Tomography (PET) or Single-Photon Emission Computed Tomography (SPECT). Such paramagnetic labels include, but are not limited to compounds containing paramagnetic ions of Aluminum (Al), Barium (Ba), Calcium (Ca), Cerium (Ce), Dysprosium (Dy), Erbium (Er), Europium (Eu), Gandolinium (Gd), Holmium (Ho), Iridium (Ir), Lithium (Li), Magnesium (Mg), Manganese (Mn), Molybdenum (M), Neodymium (Nd), Osmium (Os), Oxygen (O), Palladium (Pd), Platinum (Pt), Rhodium (Rh), Ruthenium (Ru), Samarium (Sm), Sodium (Na), Strontium (Sr), Terbium (Tb), Thulium (Tm), Tin (Sn), Titanium (Ti), Tungsten (W), and Zirconium (Zi), and particularly, Co⁺², CR⁺², Cr⁺³, Cu⁺², Fe⁺², Fe⁺³, Ga⁺³, Mn⁺³, Ni⁺², Ti⁺³, V⁺³, and V⁺⁴, positron emitting metals using various positron emission tomographies, and non-radioactive paramagnetic metal ions.

In one embodiment of the invention, a sample is a biological sample. Examples of biological samples include, but are not limited to, tissue lysates and extracts prepared from diseased tissues, body fluids, preferably blood, more preferably serum, plasma, synovial fluid, bronchoalveolar lavage fluid, sputum, lymph fluid, ascites, urine, amniotic fluid, peritoneal fluid, cerebrospinal fluid, pleural fluid, pericardial fluid, and alveolar macrophages.

In one embodiment of the invention, the term "sample" is intended to mean a sample taken from an individual prior to any analysis.

The technical solutions of the present invention will be specifically described below by examples, which are descriptive and illustrative, and are not meant to be limiting. The reagents used in the following examples, if not specifically noted, can be easily purchased from reagent companies such as Sigma Aldrich and Merck, and the test methods, if not specifically noted, can be found in textbooks such as Sambrook, J., Fritsch, EF and Maniatis, T. (1989) Molecular Cloning: A Laboratory Manual. Cold Spring Harbor Press, New York.

### Examples

### Example 1 Affinity maturation of the antibody W20

Aβ oligomers (as to the preparation method, please see CN101463082A) were diluted to 10-100 µg/mL with coating buffer (PBS, pH=7.4), 4mL of the mixture obtained were taken and added to an immunotube, and coated at 4°C overnight. The supernatant was discarded and the tube was quickly washed 3 times with PBS. The immunotube was filled with 3% bovine serum albumin (BSA) and blocked vertically at room temperature for 2h. The supernatant was discarded and the tube was quickly washed 3 times with PBS. The phage antibody library (W20 random mutation library, which is a phage antibody library constructed by introducing random mutations in the antibody CDR regions using error-prone PCR and efficiently transforming *Escherichia coli*) was suspended in 4mL 3% BSA and added to the immunotube, incubated upside down at room temperature for 1h, and then incubated vertically for 1h. Then washing 10 times with PBS containing 0.1% Tween-20 (washing 20 times in the second round of screening and thereafter). After PBS was drained, 500 µL of glycine solution (0.1M, pH=7.4) was added to elute the phages, and incubated upside down at room temperature for 10 min. 250 µL of the eluted phages were added to 1.75 mL of *E. coli* TG1 (purchased from the MRC Center, UK) with an OD₆₀₀ = 0.4, and incubated at 37 °C for 30 min statically. The remaining eluted phages were stored at 4 °C. 10⁴-, 10⁶-, and 10³-times dilutions of TG1 culture medium were spread on 2xTY plates and cultured at 37 °C overnight. The remaining TG1 culture medium was centrifuged at 11,600g for 5 min at 4 °C, and the precipitate was resuspended with 100 µL of culture medium, spread on 2xTY plates, and cultured at 37 °C overnight. The input-output ratio was calculated by counting the number of colonies grown on 2xTY plates (input-output ratio = phage antibody titer after enrichment/phage antibody titer before enrichment).

2 mL of 2xTY medium were added to the plate full of bacterial colonies, the bacteria were scraped with a glass rod, the bacterial suspension was collected, 50 µL were taken and added to 50 mL of 2xTY medium, ampicillin (Sigma, product cat. A9518) with a final concentration of 100 µg/mL and 1% glucose were added to the media, and shaked at 37°C until OD₆₀₀ = 0.4. 15% glycerol were added to the remaining bacterial solution and stored at-70°C. 1x10¹¹ helper phage M13K07 (purchased from the MRC Center, UK) was added to 10 mL of culture, incubated at 37°C for 30 min, and the bacterial solution was centrifuged at 3,000g at 4°C for 10 min, resuspended in 50 mL of 2xTY medium, and Amp with a final concentration of 100µg/mL, 50µg/mL kanamycin (Kana) and 0.1% glucose were added, and cultured overnight at 30°C in a shaking incubator. The overnight culture was centrifuged at 3,300g at 4°C for 15 min, the supernatant was collected, 1/4 volume of PEG (polyethylene glycol, PEG-6000) (20%) and NaCl (2.5 M) mixed solution was added, and the mixture was placed at 4° C for more than 1 hour after being fully mixed. After centrifugation at 3,300g at 4°C for 30 min, the supernatant was fully discarded, and the pellet was resuspended in 2 mL of PBS. Centrifuging at 11,600g for 10 min at 4°C, and the supernatant obtained is the phage antibody display library after the first round of enrichment. After each screening, 1 µL of phage supernatant was taken to determine the titer. Specifically, the phage stock solution was gradient diluted, infected with E. *coli* at 37°C for 30 min, and then spread on 2xTY plates and cultured at 37°C overnight. The clones grown on the 2xTY plate were counted, and the number of phage clones per unit volume of the phage stock solution was calculated by the dilution ratio. The above enrichment and screening process was repeated 3 times, that is, the second, third, and fourth rounds of screening were performed.

**Table 5 Screening of phage antibody library against Aβ oligomers APO*W20**

| Number of screening rounds | First round | Second round | Third round | Fourth round |
|---|---|---|---|---|
| Input (pfu) | 1.0×10¹² | 1.0×10¹² | 1.1×10¹² | 1.3×10¹² |
| Output (pfu) | 7.0×10⁷ | 1.5×10⁸ | 1.0×10⁸ | 2.4×10¹⁰ |
| Output-input ratio | 7.0×10⁻⁵ | 1.5×10⁻⁴ | 9.1×10⁻³ | 1.8×10⁻² |

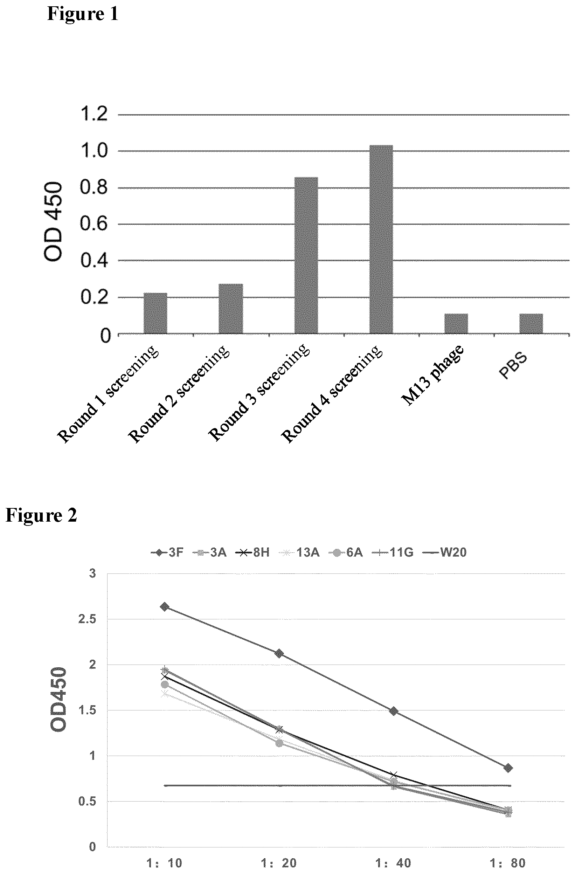

The results were shown in Table 5. After four rounds of screening, the input-output ratio of phage antibodies gradually increased. The input-output ratio of the fourth round was more than 200 times that of the first round, indicating that phages displaying high-affinity antibodies against Aβ oligomers were effectively enriched. At the same time, polyclonal ELISA analysis was performed on the four rounds of antibody libraries in the screening process. Specifically, the binding ability of the phage library obtained in each round with Aβ oligomers was determined by ELISA. The experimental principle is to coat Aβ oligomers on the ELISA plate, then to add the phage library (1E10 cfu) to bind to it, and then to detect it by indirect ELISA using horseradish peroxidase (HRP)-coupled anti-M13 phage antibodies. The results were shown in Figure 1, wherein the M13 phage group was the negative control, and the PBS group was the blank control. It can be seen from the Figure that from the first round of phage antibody library to the fourth round of phage antibody library, their binding ability with Aβ oligomers showed a gradual upward trend and was higher than the negative control group.

### Example 2 ELISA Identification of Single-chain Antibody Monoclones

The phages that had undergone the above four rounds of enrichment screening were infected with *E. coli* HB 2151 (purchased from the MRC Center, UK) and spread on plates for incubation. Single colonies were picked and placed in wells of 96-well cell culture plates. 200 µL of 2xTY medium containing 100 µg/mL Amp and 1% glucose was added to each well. The culture was cultured overnight at 37°C shaker (300r/min). 2 µL of bacterial fluid was pipetted from each well and added to another new 96-well bacterial culture plate. 200 µL of 2xTY medium containing 100 µg/mL Amp and 1% glucose was added to each well. The culture was cultured at 37 °C shaker. After OD₆₀₀ reached 1.0, glycerol was added to the first plate to a final concentration of 15%, and stored at -70 °C. IPTG was added to each well of the second 96-well plate to a final concentration of 1mmol/L, and cultured overnight at 30 °C shaker. Through ELISA analysis of more than 300 colonies, 6 dominant mutants (positive clones) were selected for gradient ELISA. Specifically, the binding ability of monoclonal phage (1E10 cfu) to Aβ oligomers was determined by ELISA. The experimental principle is to coat Aβ oligomers on ELISA plates, then to add monoclonal phages to bind to them, and then to detect and analyze them by indirect ELISA using HRP-coupled anti-M13 phage antibodies. Specifically, 4 gradient dilutions were selected for the 6 dominant mutants, which were 1:10, 1:20, 1:40 and 1:80, respectively, and the antibody W20 (1:10) was used as a control. The experiment was repeated three times and the average value was taken. The results were shown in Figure 2. It can be seen that the affinity of the 6 dominant mutants has been greatly improved, especially the antibody 3F, which is still higher than that of the antibody W20 diluted 1:10 under 1:80 dilution, and the affinity is significantly improved.

### Example 3 Single-chain antibody sequence determination

The positive clones above were sequenced and analyzed using the sequencing primer LMB3: 5' CAGGAAACAGCTATGAC 3' (SEQ ID No. 2), pHEN seq: 5' CTATGCGGCCCCATTCA 3' (SEQ ID No. 3). The clones that conform to the basic structure of the antibody in the antibody library are complete single-chain genetic engineering antibodies. The sequences measured are as follows: SEQ ID Nos. 4-9, wherein the heavy chain and light chain CDR sequences of the antibody 3F are shown in SEQ ID Nos. 10-15, respectively.

### Example 4 Single-chain antibody sequence alignment

The sequences of the above positive clones were aligned with the antibody W20 using the ClustalW software, and the results were shown in Figure 20. The amino acid numbering of the antibodies is the position of the single-chain antibody where the amino acid is located, and wherein the corresponding relationship between the light chain CDR3 region and the Kabat numbering system is: (223, L89), (224, L90), (225, L91), (226, L92), (227, L93), (228, L94), (229, L95), (230, L96), (231, L97). P228 in the antibody W20 mutated to R228 in all mutant sequences, indicating that amino acid position 228 is crucial for the binding of the single-chain antibodies to Aβ oligomers (amino acid numbering adopts the Kabat numbering system, the same below). By aligning the sequences of the antibody 13A and the antibody 3F, it was found that the affinity of the single-chain antibody was further improved after G226S227 was replaced by A226V227. Since the properties of glycine at position 226 are similar to those of alanine, it is suggested that the amino acid at position 227 located in the light chain CDR3 is an important site for the binding of the single-chain antibody to Aβ oligomers. In addition, by aligning the sequences of the antibodies 6A, 8H, and 3A, it can be found that when V226 was mutated to a more hydrophobic amino acid residue such as W226 or F226, their affinity to Aβ oligomers is further enhanced. The above results suggest that the amino acid at position 226 located in the light chain CDR3 in the single-chain antibody sequence plays an important role in the binding process of the single-chain antibody to Aβ oligomers. The above results indicate that the fragment (N/Q) 224- (S/T) 225-X226-X227-X228 (SEQ ID No. 16) in the light chain CDR3 sequence of the antibody (wherein X226 is any one of glycine, valine, leucine, isoleucine, phenylalanine, tryptophan or proline; X227 is any one of phenylalanine, tryptophan, tyrosine, alanine, leucine or isoleucine; X228 is any one of alanine, valine, leucine, isoleucine, methionine, aspartic acid, glutamic acid, lysine, glycine, serine, threonine, cysteine, asparagine or glutamine) is the key site for the binding of the single-chain antibody to Aβ oligomers.

### Example 5 Molecular simulation of single-chain antibody structure

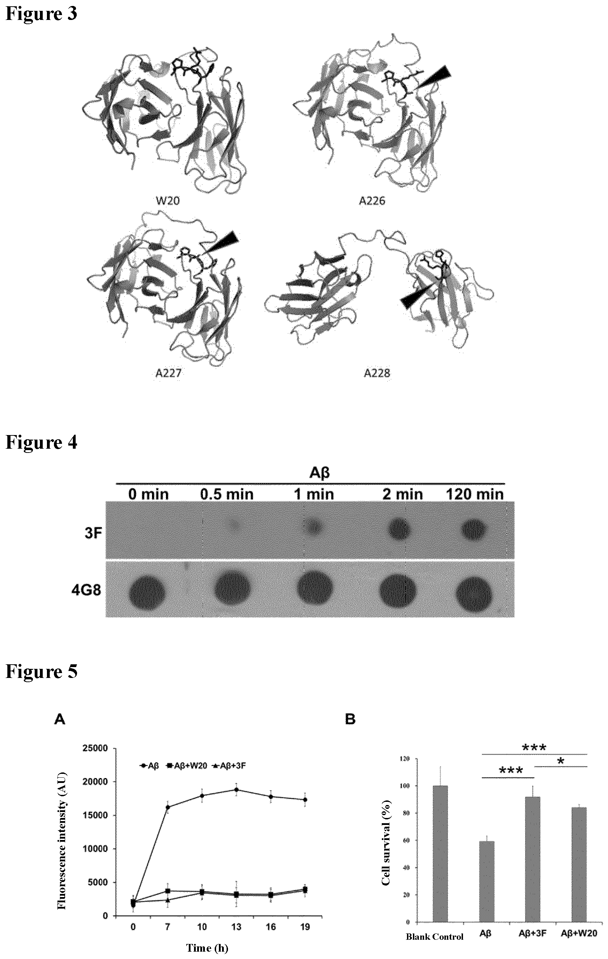

The original pdb file was established in this example by the homology modeling method on the basis of the single-chain antibody sequences; based on the original pdb file, water and Cl⁻ and Na⁺ for adjusting the pH value were added, and the molecular dynamics simulation program was run to obtain the pdb file at human room temperature (298 K); based on the pdb file at room temperature, the molecular dynamics software Gromacs was used to calculate the full atomic structure of the single-chain antibody protein. The amino acid residues at positions 226, 227, and 228 of the single-chain antibody W20 were mutated to alanine, and then the single-chain antibody structure is calculated by molecular simulation, and the results are shown in Figure 3. By analyzing the CDR3 region, it is found that after alanine mutation at position 226, the side chain residue orientation of the arginine residue at position 227 of the single-chain antibody changes, and after alanine mutation at position 227, the structure does not change significantly. It can be seen from the conclusion of Example 4 that the amino acid residues at positions 226 and 227 are key residues for binding to Aβ oligomers. In this example, the antibody structure analysis shows that the single-chain antibody binds to the Aβ oligomer through the amino acid residue at position 227, and the amino acid residue at position 226 can affect the orientation of the amino acid residue at position 227, thus having an important influence on the binding. After the alanine mutation at position 228, the structure of the single-chain antibody changes significantly, indicating that the amino acid residue at position 228 plays an important role in maintaining the structure of the single-chain antibody.

### Example 6 Specific binding of the antibody 3F to Aβ oligomers

Dot blot experiments were used to evaluate the binding of antibodies to Aβ42 monomers and Aβ oligomers. Aβ samples incubated for 0, 0.5 min, 1 min, 2 min, and 120 min were spotted on nitrocellulose membranes and blocked with 5% skim milk for 1 h at room temperature. The membranes were incubated with the detection antibodies 3F or 4G8 (Biolegend, Cat. No. 800704) at room temperature for 1-2 h, then washed three times with 0.1% TBST for 5 min each, and HRP-labeled anti-c-myc secondary antibodies (Santa Cruz, Cat. No. H2317) or goat anti-mouse secondary antibodies (Abcam, Cat. No. ab6789) were added and incubated for 1 h at room temperature. The membrane was washed three times with 0.1% TBST for 5 min each time, and ECL chemiluminescent solution was added to the membrane. The membrane was analyzed using the Amersham Imager 680 imaging system. The results showed that the antibody 3F did not bind to Aβ monomers but mainly bound to Aβ oligomers (Figure 4).

### Example 7 Determination of affinity constant K_{D} of antibodies

Surface plasmon resonance (SPR) assay was used to measure the K_{D} of antibodies. The antibody 3F was fixed on the detection chip (CM5) (purchased from Biacore, Sweden) and equilibrated with HBS-EP buffer overnight. Aβ oligomers were diluted with HBS-EP buffer to different concentration gradients. The loading volume was 35 µL, the flow rate was 5 µL/min, the data lag time after sample loading was 120 seconds, and the protein binding signal was detected. Finally, the BIAcore analysis software was used to read the data and calculate the K_{D} value of each antibody. The affinity constant between the antibody 3F and Aβ oligomers was measured to be K_{D}=8.47×l0⁻¹⁰ M.

### Example 8 The antibody 3F can significantly inhibit Aβ aggregation and Aβ oligomer-induced cytotoxicity

According to the method described by Liu et al. (J Nanobiotechnol (2020) 18: 160.), the effect of the antibody 3F on Aβ aggregation was evaluated by ThT fluorescence method, and the effect of the antibody 3F on Aβ oligomer-induced cytotoxicity was evaluated by MTT method. ThT fluorescence results showed that both the antibody 3F and the antibody W20 could significantly reduce Aβ aggregation, while Aβ alone continued to aggregate (Figure 5A). The results of cell MTT experiments showed that the antibody 3F could significantly inhibit the cytotoxicity of SH-SY5Y cells induced by Aβ oligomers, and its inhibitory activity was better than that of the antibody W20 (Figure 5B).

### Example 9 The antibody 3F can significantly improve the cognitive function of AD transgenic mice

Six-month-old male APP/PS1 mice (purchased from Beijing HFK Bioscience Co., Ltd.) were randomly divided into three groups, 8 mice per group, and the antibodies 3F, W20 or PBS (AD CON.) were administered intranasally once a day for 28 days; the wild-type control group mice were administered an equal amount of PBS intranasally. The spatial memory and cognitive ability of mice were tested according to the water maze and Y maze experimental methods described by Yu et al. (Br J Pharmacol. 2020; 177: 2860-2871.). The results showed that compared with the AD CON. group, the time for APP/PS 1 mice treated with the antibody 3F to reach the platform during the training period was significantly shortened (Figure 6, panel A). During the exploration period after the platform was removed, APP/PS1 mice in the antibody 3F group showed obvious spatial directional swimming behavior, and the number of crossing the platform increased significantly (Figure 6, panel B). Similarly, in the Y maze experiment, mice in the AD CON. group did not have a clear preference for the new arm. The time that the APP/PS1 mice in the antibody 3F group stayed in the new arm increased significantly (Figure 6, panel C). This indicates that the antibody 3F can significantly improve the cognitive function of AD transgenic mice. Moreover, compared with the antibody W20, the antibody 3F has a more significant ability to improve the cognition of AD transgenic mice.

### Example 10 The antibody 3F can significantly reduce the level of Aβ in the brains of AD transgenic mice

Using the antibody 4G8 as the primary antibody, immunohistochemistry was used to detect the level of Aβ plaques in the brains of APPB/PS 1 mice. Compared with the mice in the AD CON. group, the plaque area in the cortex and hippocampus of APPB/PS1 mice treated with the antibody 3F was significantly reduced (Figure 7). ELISA was further used to detect the content of Aβ40/42 in mouse brain homogenate. The results showed that the antibody 3F could significantly reduce the levels of Aβ40 and Aβ42 in the brains of APPB/PS1 mice (Figure 8). Moreover, compared with the antibody W20, the antibody 3F could more significantly reduce the levels of plaques and Aβ40/Aβ42 in the brains of APPB/PS1 mice.

### Example 11 The antibody 3F can significantly reduce the degree of glial cell activation and inflammatory factor levels in the brains of AD transgenic mice

The main characteristics of neuroinflammation include excessive activation of glial cells and massive release of inflammatory factors. Iba-1 immunostaining and GFAP immunostaining were used to detect the activation of microglia and astrocytes in the mouse brain. The results showed that compared with the mice in the AD CON. group, the Iba-1 and GFAP positive staining areas in the cortex and hippocampus of the APP/PS1 mice treated with the antibody 3F were significantly reduced (Figure 9), indicating that the antibody 3F can significantly reduce the activation of glial cells in the brains of AD transgenic mice. Furthermore, ELISA was used to determine the content of IL-1β and IL-6 in mouse brain homogenates. Compared with the mice in the AD CON. group, the level of inflammatory cytokines in the brains of the APP/PS1 mice treated with the antibody 3F was significantly reduced (Figure 10). Moreover, compared with the antibody W20, the antibody 3F has a more significant ability to reduce the level of neuroinflammation in the brains of AD transgenic mice.

### Example 12 The antibody 3F can significantly improve the behavioral coordination ability and cognitive function of PD transgenic mice

Twelve-month-old male A53T α-synuclein transgenic mice (purchased from Jackson Lab) were randomly divided into three groups, 8 mice per group, and the antibodies 3F, W20 or PBS (PD CON.) were administered intranasally once a day for 28 days; wild-type control group mice were given an equal amount of PBS intranasally. The pole climbing test was used to detect the motor coordination ability of mice.

Pole climbing test: The pole used is a wooden pole with a rough surface and a base, 1 cm in diameter and 50 cm in length. The pole was placed steadily in the mouse cage, and the mouse was placed on the top of the pole with its head facing up, so that it naturally turns its head and then climbs down the pole into the cage. The time it takes for the mouse to turn its head and climb into the cage was recorded. If the mouse falls, slips, or fails to complete the task, the time to turn its head is recorded as 30 seconds, and the time to climb back into the cage is recorded as 60 seconds. This was done 5 times a day, with the first two days being the training period and the third day being the test period. The time it takes for each group of mice to turn their heads and climb into the cage during the test period was statistically analyzed.

The results showed that the time taken by A53T α-synuclein transgenic mice treated with the antibody 3F to turn their heads and climb into the cage was significantly shorter than that of mice in the PD CON group. In addition, the time taken by mice in the antibody 3F group was shorter than that in the antibody W20 group (Figure 11, panels A and B).

Novel object recognition experiment was designed based on the nature of mice to explore new things. The device was a white box with a size of 40 cm x 40 cm x 40 cm. The experiment was divided into three stages: adaptation stage, training stage and detection stage. In the adaptation stage, mice were placed in an empty box to spontaneously adapt for 5 min. After 24 h, the training stage began. Two identical objects were placed in the box to allow mice to get familiar in the box for 5 min. After an interval of 6 h, the detection stage began. One of the old objects in the box was replaced with a new object. The mice were allowed to explore the box freely for 5 min, and the number of times the mice explored the new and old objects was recorded. The cognitive index was determined by the following calculation formula: (number of new objects - number of old objects)/(number of new objects + number of old objects). In order to avoid the influence of mouse odor, the box was wiped with 75% alcohol after each mouse finished exploring.

The results showed that the antibody 3F-treated A53T α-synuclein transgenic mice had significantly higher cognitive ability for novel objects than the PD CON group mice. Moreover, compared with the antibody W20 group mice, the antibody 3F group mice also had stronger cognitive ability for novel objects (Figure 11, panel C).

### Example 13 The antibody 3F can significantly reduce the level of α-synuclein in the brains of PD transgenic mice

Phosphorylated Ser129 α-synuclein (pSer129-α-syn) is a specific pathological form of α-synuclein in the brains of PD patients. The inventors used anti-pSer129-α-syn antibody (Abcam, Cat. No. ab59264) as the primary antibody and used immunohistochemistry to detect the level of pathological α-synuclein in the brains of A53T α-synuclein transgenic mice. There were obvious pSer129-α-syn positive areas in the brainstem of PD CON. group mice, while the pSer129-α-syn positive area in the brainstem of A53T α-synuclein transgenic mice treated with the antibody 3F was significantly reduced (Figure 12). Moreover, compared with the antibody W20, the antibody 3F can more significantly reduce the level of pSer129-α-syn in the brainstem of A53T α-synuclein transgenic mice.

### Example 14 The antibody 3F can significantly increase the level of tyrosine hydroxylase in the brains of PD transgenic mice

Tyrosine hydroxylase (TH) is the rate-limiting enzyme for dopamine synthesis and plays an important role in dopamine signal transduction. The expression of TH is negatively correlated with the severity of PD. The inventors used immunohistochemistry to detect the level of TH in the brains of A53T α-synuclein transgenic mice. The results showed that compared with WT mice, the expression level of TH in the brainstem of A53T α-synuclein transgenic mice was significantly reduced, while the expression of TH in the brainstem of PD mice treated with the antibody 3F was significantly increased (Figure 13). Compared with the antibody W20, the antibody 3F can more significantly increase the level of TH in the brains of PD transgenic mice.

### Example 15 The antibody 3F can significantly improve the spontaneous activity ability and anxiety behavior of HD transgenic mice

Five-week-old male R6/2 mice (purchased from JACKSON LAB) were randomly divided into three groups, 8 mice per group, and the antibodies 3F, W20 or PBS (PD CON.) were administered intranasally once a day for 28 days; the wild-type control group mice were given an equal amount of PBS intranasally. The open field test was used to detect the motor coordination ability of the mice.

The open field test is a method to evaluate the autonomous behavior, exploratory behavior and tension of experimental animals in a novel environment. The open field is a white open plastic box (27 cm x 27 cm x 20.3 cm), which is evenly divided into 9 areas in the form of a nine-square grid. A camera is installed on the top of the box to capture the movement trajectory of the mice. The mice were placed in the open field one at a time. After 1 min of adaptation, the movement trajectory of the mice was recorded by camera for 30 min. The inner walls of the box were cleaned with 70% ethanol before the experiment for each mouse. The following three indicators were quantitatively analyzed: total distance, number of standing times in all areas, and time spent in the central area.

The experimental results showed that compared with WT mice, mice in the HD CON. group showed a significant decrease in autonomous activity ability and obvious anxiety symptoms, including a decrease in the total distance of movement, the number of hind limb standing, and the time spent in the center area in the open field. The treatment with the antibody 3F can significantly increase the total distance of activity, the number of standing, and the time spent in the center area of R6/2 mice (Figure 14). Moreover, compared with the antibody W20, the antibody 3F can more significantly improve the autonomous activity ability of R6/2 mice and alleviate their anxiety state.

### Example 16 The antibody 3F can significantly reduce the level of mHTT aggregates in the brains of HD transgenic mice

In the brains of HD patients or HD transgenic animals, the massive aggregation and deposition of mHTT protein in the nuclei and cytoplasm of neurons is an important pathological feature. EM48 antibody (Merck Millipore, Cat. No. MAB5374) can specifically identify mHTT aggregates in pathological states. Immunofluorescence staining of R6/2 mouse brain tissue using EM48 antibody showed that there was obvious EM48 positive staining in the striatum and cortex of the brains of HD CON. group mice. After the treatment with the antibody 3F, the EM48 positive area in the brains of R6/2 mice was significantly reduced (Figure 15), indicating that the treatment with the antibody 3F effectively reduced the mHTT level in the brains of HD transgenic mice, and its effect was better than that of the antibody W20.

### Example 17 The antibody 3F can significantly improve the motor function of ALS transgenic mice

Eleven-week-old male SOD1-G93A transgenic mice (purchased from Model Animal Research Center, Nanjing University, China) were randomly divided into three groups, 8 mice per group. The antibodies 3F, W20 or PBS (ALS CON.) were administered intranasally once a day for 28 days. The wild-type control group was given an equal amount of PBS intranasally. The motor coordination ability of the mice was tested by the hanging test and the rotarod test.

Hanging test: The mouse was hanged on the cage cover, the cage cover was turned over and the mouse was placed 50 cm away from the cage, observed for 60 seconds, and the time the mouse stays on the cage cover was recorded. If the mouse stays for more than 60 seconds, it will be recorded as 60 seconds.

The results showed that compared with the ALS CON group mice, the SOD1-G93A mice treated with the antibody 3F were able to maintain a significantly longer time in the hanging test, indicating that the antibody 3F can effectively enhance the muscle strength of mice (Figure 16, panel A). Moreover, the effect of the antibody 3F was superior to that of the antibody W20.

Rotarod test: The rotarod test was conducted for 3 consecutive days, and each day consisted of two phases: training phase and testing phase. Training phase: The rotation speed of the rotarod was 4 rpm. The mice were put back into the cage after 5 minutes of training on the rotarod, and entered the testing phase 1 hour later. Testing phase: The rotation speed of the rotarod was uniformly accelerated from static to 40 rpm within 5 minutes, and the duration of the mouse on the rotarod was recorded. 300 seconds will be recorded if the mice did not fall. The test was conducted 3 times a day, with an interval of 30 minutes each time. Each group of mice was tested a total of 9 times in 3 days, and the duration of each time on the rotarod was statistically analyzed.

The results showed that the duration of ALS CON. group mice on the rotarod was significantly lower than that of WT mice, while the duration of SOD1-G93A mice in the antibody 3F treatment group on the rotarod was significantly increased, and the duration of the antibody 3F group mice on the rotarod was also longer than that of the antibody W20 group mice (Figure 16, panel B).

### Example 18 The antibody 3F can significantly reduce the level of SOD1 aggregates in the brainstem of ALS transgenic mice

There are massive mutant SOD1 aggregates deposited in the brainstem of ALS patients and transgenic mice. The inventors performed SOD1 immunostaining on brain sections of SOD1-G93A mice. The results showed that compared with ALS CON. group mice, the SOD1 aggregates in the brainstem of SOD1-G93A mice in the antibody 3F treatment group were significantly reduced (Figure 17). Moreover, compared with the antibody W20, the antibody 3F can more effectively reduce the level of SOD1 aggregates in the brainstem of ALS transgenic mice.

### Example 19 The antibody 3F can significantly reduce the activation level of glial cells in the brains of ALS transgenic mice

The present inventors evaluated the activation level of microglia and astrocytes in the brains of SOD1-G93A mice by immunostaining of Iba-1 and GFAP. The results showed that compared with WT mice, the activated glial cells in the brains of ALS CON. group mice increased significantly, and the treatment with the antibody 3F could significantly reduce the activation level of microglia and astrocytes in the brains of SOD1-G93A mice. Compared with the antibody W20, the antibody 3F has a better ability to reduce the activation of glial cells in the brains of ALS transgenic mice (Figure 18).

### Example 20 Improved antibodies such as the antibody 3F can significantly improve the cognitive function of AD transgenic mice

Six-month-old male APP/PS1 mice (purchased from Beijing HFK Bioscience Co., Ltd.) were randomly divided into 8 groups, 8 mice per group, and the antibodies 3F, W20, 3A, 6A, 8H, 11G, 13A or PBS (AD CON.) were administered intranasally once a day for 28 days; the wild- type control group mice were administered an equal amount of PBS intranasally. The cognitive function of mice was tested according to the Y-maze experimental method described by Yu et al. (Br J Pharmacol. 2020; 177: 2860-2871.). The results showed that the mice in the AD CON. group had no obvious preference for the new arm. The time that APP/PS 1 mice in each antibody group stayed in the new arm increased significantly (Figure 19). Among them, the antibody 3F had the most significant ability to improve the cognition of AD transgenic mice.

### Example 21 Specific Binding of the Antibody 3FI4 (IgG4 Subtype of the antibody 3F) to Aβ Oligomers

The present invention transformed the antibody 3F into IgG4 subtype antibody with conventional methods, named the antibody 3FI4 (3FI4-1, 3FI4-2, 3FI4-3, 3FI4-4, of which the light chain sequence is all shown in SEQ ID No. 17, the heavy chain sequences are shown in SEQ ID Nos. 18-21, the light chain constant region sequence is shown in SEQ ID No. 22, and the heavy chain constant region sequences are shown in SEQ ID Nos. 23-26). The binding ability of the antibodies to Aβ oligomers is determined by ELISA method. Specifically, Aβ oligomers were coated at 0.2 µg/well overnight at 4 °C. The next day, the diluted 3FI4 antibodies were added to the coated ELISA plate at 100 µL/well, incubated at 37 °C for 1 h; washed 3 times with PBST, and the residual PBST was patted dry; 1: 10,000 diluted goat anti-human HRP secondary antibody was added at 100 µL/well, incubated at 37 °C for 1 h, washed 3 times with PBST, and the residual PBST was removed. TMB chromogenic solution was added at 100 µL/well, color was developed at 37 °C or room temperature, the reaction was terminated with 2 M hydrochloric acid, and the absorbance value at OD₄₅₀ nm was detected by an enzyme reader (Figure 21). The results showed that the antibody 3FI4 could specifically bind to Aβ oligomers.

### Example 22 The Aβ oligomer Aβo*3F specifically bing to the antibody 3F has extremely strong toxicity

### 22.1 Aβ oligomers that specifically bind to the antibody 3F

### 22.1.1 In vitro preparation of the Aβo*3F

1 mg of Aβ42, Aβ40 or other forms of Aβ (Chinese Peptide Co., Ltd.) was dissolved in 1 mL of 100% hexafluoroisopropanol (HFIP), vortexed for 5 min, and ultrasonicated in a water bath for 10 min, then dispensed into EP tubes, the solvent was evaporated overnight, and stored at -20 °C. Before use, the HFIP-treated and aliquoted Aβ was dissolved in 50 mM NaOH at a concentration of 1 mg/mL, vortexed for 3-5 min, ultrasonicated for 1 min, and then diluted to 10 µM with pre-cooled PBS. Centrifuged at 21,000 g for 30-40 min at 4 °C, discarded the precipitate (about 5% of the starting volume), and Aβ monomers were obtained. The Aβ monomers were statically incubated at 25 °C for 2 days, and then incubated with Protein A magnetic beads cross-linked with the antibody 3F at 4 °C overnight. On the next day, the magnetic beads were washed three times with 0.1% PBST and eluted twice with 20-100 mM glycine (pH 2.0) for 3-5 min. The eluate was neutralized to pH7 with 1 M Tris to obtain sAβo*3F (Aβos prepared *in vitro*)*.*

### 22.1.2 Isolation and preparation of the Aβo*3F from APP/PS1 mouse brain homogenate and AD patient CSF

To isolate and prepare Aβos specifically recognized by the antibody 3F (Aβo*3F), brain homogenates of APP/PS1 mice or CSF samples of AD patients (obtained from the First Affiliated Hospital of Zhengzhou University, with informed consent and approval from the Ethics Review Committee of the First Affiliated Hospital of Zhengzhou University) were incubated with Protein A magnetic beads cross-linked with the antibody 3F at 4°C overnight. The next day, the magnetic beads were washed three times with 0.1% PBST and then eluted twice with 20 mM-100 100 mM glycine (specifically 20 mM, pH2.0) for 3 min, and the eluate was neutralized to pH7 with 1 M Tris, and then the Aβo*3F in the brains of APP/PS1 mice (mAβo*3F, the Aβo*3F isolated from the brains of AD mice) or the Aβo*3F extracted from the CSF of AD patients (hAβo*3F, the Aβo*3F isolated from the cerebrospinal fluid of AD patients) was obtained. The Aβ aggregate mixture after the antibody 3F immunodepletion is called Aβ-ID, which are sAβ- ID (prepared *in vitro*)*,* mAβ- ID (isolated and prepared from the brains of APP/PS1 mice) and hAβ- ID (isolated and prepared from the CSF of AD patients) and used as controls.

The Aβ aggregate mixture Aβ*6E10 was prepared by mixing APP/PS1 mouse brain homogenate or AD patient CSF with Protein G magnetic beads cross-linked with the 6E10 antibody, reacting at 4°C overnight, and then eluting twice with 20 mM-100 mM glycine (specifically 20 mM, pH 2.0) for 3-5 min (specifically 3 min), and the eluate was neutralized to pH7 with 1 M Tris, and then Aβo*6E10 in the brain of APP/PS1 mice (mAβo*6E10, Aβo*6E10 isolated from the brains of AD mice) or Aβo*6E10 extracted from the CSF of AD patients (hAβo*6E10, Aβo*6E10 isolated from the CSF of AD patients) was obtained (Figure 22). The concentration of Aβ obtained by immunoprecipitation was measured using an Aβ detection kit.

### 22.2 Characterization of the Aβo*3F's molecular weight and morphology

500 µL of the Aβo*3F isolated from APP/PS1 mouse brain (mAβo*3F) or 100 µL of SEC Marker was loaded onto a Superdex 200 10/300GL molecular exclusion chromatography column connected to an AKTA pure system. The column was pre-equilibrated with Tris-Gly buffer and eluted at a flow rate of 0.5 mL/min. The molecular weight of mAβo*3F was found to be 588 kDa by comparison with the Marker (Figure 23, panel A). In addition, 10 µL of mAβo*3F was dropped onto a 200-mesh copper grid and adsorbed for 20 min, blotted with filter paper, negatively stained with 2% uranyl acetate for 30s, blotted with filter paper, and air-dried. The samples were examined under a transmission electron microscope (TEM, Hitachi H7700, Japan) at a working voltage of 120 kV at 100,000 × magnification. The results showed that mAβo*3F was a particle with a diameter of about 10 nm (Figure 23, panel A). The molecular weights and distributions of sAβo*3F and Aβo*3F extracted from CSF of AD patients (hAβo*3F) were consistent with the results of mAβo*3F.

### 22.3 Strong cytotoxicity of the Aβo*3F

MTT results showed that the half inhibitory concentration (IC50) of sAβo*3F on N2a cytotoxicity was about 0.186 nM, while the IC50 of sAβos before immunoprecipitation was about 63.26 nM, with a 340-fold difference between the two IC50s (Figure 23, panels B and C). MTT assay showed that the IC50 of mAβo*3F on N2a cells was about 0.111 nM; the IC50 on primary neuronal cells was about 0.057 nM (Figure 23, panel D). MTT assay showed that the IC50 of hAβo*3F on primary neurons was about 0.076 nM (Figure 23, panel E). These results show that the Aβo*3F is an oligomer with strong neurotoxicity.

### 22.3 The Aβo*3F caused a significant decline in cognitive function in mice and severely damaged the function of neurons in the mouse brain

Three-month-old C57BL/6 mice were randomly divided into 6 groups, 6-8 mice per group, and injected intracerebroventricularly into the following groups: 2.5 nM (45.5 pg) mAβo*3F group, 2.5 nM (45.5 pg) mAβ*6E10 group, 2.5 nM (45.5 pg) mAβ-ID group, 600 nM (10.9 ng) mAβ*6E10 group, 600 nM (10.9 ng) mAβ-ID group, and a control group injected with Tris-Gly solvent. The behavior and cognitive ability of the mice were tested 24 hours after the injection, and the mice were dissected for brain pathological analysis.

The Y-maze results showed that the duration of mice treated with 2.5 nM mAβo*3F and 600 nM mAβ*6E10 in the new arm was significantly reduced compared with the control mice. However, there was no significant difference between the mice injected with 2.5 nM mAβ*6E10, 2.5 nM mAβ*ID and 600 nM mAβ*ID and the control group. This indicates that mAβo*3F, but not mAβ*6E10 or mAβ-ID, can cause severe memory impairment in mice at low concentrations. For mAβ*6E10, only when the concentration is increased to 600 nM can mice show a similar degree of memory impairment (Figure 24, panel A).

The brain samples were stained by using the FD Fast Golgi Staining Kit. By calculating the dendritic spine density, it was found that compared with the control group, the dendritic spine density of neurons in the CA1 region of the mouse brain treated with 2.5 nM mAβo*3F and 600 nM mAβ*6E10 was significantly decreased, but 2.5 nM mAβ*6E10, 2.5 nM mAβ-ID or 600 nM mAβ-ID had no significant effect on the dendritic spine density of mouse brain neurons (Figure 24, panels B, and C).

### Example 23 Further Optimization and Activity Determination of the antibody 3F

The amino acid residue K at position 100 of the antibody 3F was mutated to R (the position 98 in the antibody numbering when not considering the MA residues introduced for expression as a single-chain antibody, and the variant was named K98R mutant, and the sequence thereof is shown in SEQ ID NO: 27), and the effect of K98R mutation on the affinity of the single chain antibody was studied. The method used is as follows.

The above Aβ oligomers were coated to 96-well plates at 100 ng/well overnight at 4°C. The next day, 3% BSA was used to block for 2 h at room temperature, washed twice with PBST, and patted dry for use. The K98R mutant and the antibody 3F were diluted, starting from 1 µg/ml and 2x dilution for 14 gradients. The diluted antibody solutions were added to the ELISA plates coated with the above Aβ oligomers, and parallel replicate wells were made. Incubated at 37°C for 1 h, washed 3 times with PBST, and patted dry. 1: 10,000 diluted goat anti-human IgG-HRP, was added incubated at 37°C for 45 min, washed 3 times with PBST, and patted dry. TMB chromogenic solution was added for color development for about 15 minutes. After the stop solution was terminated, the OD was read at 450 nm, and the values were analyzed and compared using Graphpad and processed by graphing.

The results were shown in Figure 25. The experiment shows that after the amino acid residue K at position 98 of the single-chain antibody heavy chain was mutated to R, the affinity was further improved, suggesting that the amino acid residue at position 98 plays an important role in the binding process of the single-chain antibody to Aβ oligomers. The inventors considered the reasons for this result and found that when numbering according to IMGT numbering, the amino acid residue at position 98 is also the residue of the heavy chain CDR 3 of the antibody 3F (according to IMGT numbering, the heavy chain/light chain CDR sequences of the antibody 3F are: CDR-H1: GFTFSSYA (SEQ ID NO: 28); CDR-H2: ISNLGLTT (SEQ ID NO: 29); CDR-H3: AKTTSRFDY (SEQ ID NO: 30); CDR-L1: QSISSY (SEQ ID NO: 31); CDR-L2: KAS; CDR-L3: QNSAVRPVT (SEQ ID NO: 32). The inventors also studied the *in vivo* activity of the K98R variant and found that it was further improved relative to the antibody 3F (data not shown).

### Equivalent solution

Although multiple embodiments of the present invention have been described and illustrated herein, a person of ordinary skill in the art would readily envision various other means and/or structures for achieving the functions described herein and/or obtaining the results and/or one or more advantages described herein, and it is believed that each such change and/or modification is within the scope of the present invention. More broadly, it will be readily understood by those skilled in the art that all the parameters, materials and settings described herein are intended to be exemplary, and that actual parameters, materials and/or settings will depend on the specific application in which the teachings of the present invention are used. Those skilled in the art would recognize or be able to determine many equivalents of the specific embodiments of the present invention described herein using only routine experiments. Therefore, it should be understood that the foregoing embodiments and examples are presented only by way of example, and within the scope of the appended claims and their equivalents, the present invention may be implemented in a manner different from that specifically described and claimed. Any combination of two or more such features, systems, objects, materials and/or methods is included within the scope of the present invention if such features, systems, objects, materials and/or methods are not mutually conflicting.

The phrase "and/or" used in the present specification and claims should be understood to mean "either or both" of the elements so combined, i.e., elements that are present in combination in some cases and not in combination in other cases. In addition to the elements specifically identified by the "and/or" clause, other elements may optionally be present, whether related or unrelated to those specifically identified elements, unless otherwise expressly indicated. Thus, as a non-limiting example, when used in combination with open language such as "comprising", a reference to "A and/or B" may refer to A without B (optionally including other elements in addition to B) in one embodiment; to B without A (optionally including elements in addition to A) in another embodiment; to both A and B (optionally including other elements) in yet another embodiment; and so on.

As used herein in the specification and claims, "or" shall be understood to have the same meaning as "and/or" as defined above. For example, when separating items in a list, "or" or "and/or" shall be understood to be inclusive, i.e., including at least one of a plurality of elements or list of elements, but also including more than one, and optionally, other unlisted items. Only when the contrary term is clearly indicated, such as "only one of" or "exactly one of", or when used in a claim, "consisting of" will refer to comprising exactly one element of a plurality of elements or lists of elements. Generally, when preceded by an exclusive term, such as "either", "one of", "only one of" or "exactly one of", the term "or" used herein should be understood only to represent an exclusive alternative (i.e., "one or the other but not both"). "Substantially consisting of" when used in a claim shall have its ordinary meaning in the field of patent law.

As used herein in the specification and claims, when referring to a list of one or more elements, the phrase "at least one" should be understood to mean at least one element selected from any one or more elements of the list of elements, but does not necessarily include at least one of each element specifically listed in the list of elements, and does not exclude any combination of elements in the list of elements. This definition also allows that any element other than the elements specifically identified in the list of elements referred to by the phrase "at least one" may optionally be present, whether related or unrelated to those specifically identified elements. Thus, as a non-limiting example, in one embodiment, "at least one of A and B" (or equivalently, "at least one of A or B", or equivalently, "at least one of A and/or B") may refer to at least one, optionally including more than one A, without B (and optionally including elements other than B); in another embodiment, to at least one, optionally including more than one B, without A (and optionally including elements other than A); in yet another embodiment, to at least one, optionally including more than one A, and at least one, optionally including more than one B (and optionally including other elements); and so on.

In the claims and the specification, all the conjunctions, such as "comprise", "include", "with", "having", "containing", "involving", "owning", etc., are understood to be open-ended, i.e., meaning including but not limited to. Only the conjunctions "consisting of" and "consisting essentially of" shall be closed or semi-closed conjunctions, respectively.

The use of ordinal terms in the claims, such as "first ", "second", "third ", etc. to modify claim elements, does not imply any priority, precedence, or order of one claim element relative to another claim element, or a temporal order of actions in a method, but merely serves as a label to distinguish one claim element with a certain name from another element with the same name (but for ordinal terms) to distinguish claim elements.

## Claims

1. An improved amyloid protein oligomer-specific antibody or antigen-binding fragment thereof, having amino acid substitutions at one or more positions selected from the group consisting of amino acid residue positions 226, 227 and 228 relative to the antibody W20, of which the amino acid sequence is shown in SEQ ID No. 1, and the amino acid position numbering of the antibody being numbered according to the Kabat numbering system.

2. The improved amyloid protein oligomer-specific antibody or antigen-binding fragment thereof according to claim 1, wherein the amino acid residue at positions 226 is substituted with a non-polar, hydrophobic amino acid having properties similar to those of alanine, preferably, substituted with glycine, valine, leucine, isoleucine, phenylalanine, tryptophan or proline, more preferably, substituted with glycine, phenylalanine or tryptophan.

3. The improved amyloid protein oligomer-specific antibody or antigen-binding fragment thereof according to claim 1 or 2, wherein the amino acid residue at position 227 is substituted with a hydrophobic amino acid having properties similar to those of valine, preferably substituted with phenylalanine, tryptophan, tyrosine, alanine, leucine or isoleucine, more preferably substituted with phenylalanine, isoleucine or leucine.

4. The improved amyloid protein oligomer-specific antibody or antigen-binding fragment thereof according to any one of claims 1 to 3, wherein the amino acid residue at position 228 is substituted with an aliphatic amino acid having a structure similar to that of arginine, preferably, substituted with alanine, valine, leucine, isoleucine, methionine, aspartic acid, glutamic acid, lysine, glycine, serine, threonine, cysteine, asparagine or glutamine, more preferably, substituted with lysine, asparagine or glutamine.

5. The improved amyloid protein oligomer-specific antibody or antigen-binding fragment thereof according to any one of claims 1 to 4, wherein the amino acid residues at positions 226 to 228 are substituted with AVR, GSR, WVR, FER, NFR or VRR, respectively.

6. The improved amyloid protein oligomer-specific antibody or antigen-binding fragment thereof according to any one of claims 1 to 5, wherein the amino acid residues at positions 224 and 225 are substituted with glutamine and threonine, respectively.

7. The improved amyloid protein oligomer-specific antibody or antigen-binding fragment thereof according to any one of claims 1 to 6, which is an antigen-binding fragment, preferably, an antigen-binding fragment selected from the group consisting of scFv, F(ab')₂, Fab', Fab, Fd, Fv, bispecific antibody, camel antibody, CDR and antibody minimal recognition unit (dAb), more preferably, an antigen-binding fragment selected from the group consisting of scFv, F(ab')₂, Fab', and Fab.

8. The improved amyloid protein oligomer-specific antibody or antigen- binding fragment thereof according to any one of claims 1 to 6, which is a monoclonal antibody, a chimeric antibody, a humanized antibody or a fully human antibody, preferably, the antibody is selected from the group consisting of IgG, IgM, IgA, IgD, IgE and their subtypes, more preferably, the antibody is selected from the group consisting of subtypes IgG1-4, more preferably, the antibody is IgG4 subtype.

9. The improved amyloid protein oligomer-specific antibody or antigen-binding fragment thereof according to any one of claims 1 to 8, which is a scFv and has an amino acid sequence as shown in any one of SEQ ID Nos. 5, 4 and 6 to 9.

10. The improved amyloid protein oligomer-specific antibody or antigen-binding fragment thereof according to any one of claims 1 to 9, wherein the sequence of CDR3 of the light chain thereof is shown in SEQ ID No. 15.

11. The improved amyloid protein oligomer-specific antibody or antigen-binding fragment thereof according to any one of claims 1 to 9, wherein the amino acid residue at position 98 thereof relative to the antibody 3F is substituted with arginine, preferably, the heavy chain amino acid sequence of the improved amyloid protein oligomer-specific antibody or antigen-binding fragment thereof is as shown in any one of SEQ ID Nos. 33-36, preferably, the improved amyloid protein oligomer-specific antibody or antigen-binding fragment thereof is scFv and the amino acid sequence thereof is as shown in SEQ ID No. 27.

12. An isolated nucleic acid molecule selected from the group consisting of:
(1) DNA or RNA encoding the improved amyloid protein oligomer-specific antibody or antigen-binding fragment thereof according to any one of claims 1 to 11; and
(2) a nucleic acid molecule completely complementary to the DNA or RNA defined in item (1).

13. An expression vector comprising an operatively linked nucleic acid molecule according to claim 12.

14. A host cell comprising the nucleic acid molecule according to claim 12 or the expression vector according to claim 13.

15. A composition comprising the improved amyloid protein oligomer-specific antibody or antigen-binding fragment thereof according to any one of claims 1 to 11, the nucleic acid molecule according to claim 12, the expression vector according to claim 13 or the host cell according to claim 14, and one or more pharmaceutically acceptable carriers, diluents or excipients, optionally, the composition further comprises an additional neurodegenerative disease therapeutic agent, preferably, the additional neurodegenerative disease therapeutic agent is selected from the group consisting of acetylcholinesterase inhibitors such as donepezil, galantamine, carbastine, and aspartate receptor antagonists such as memantine.

16. A method for producing the improved amyloid protein oligomer-specific antibody or antigen-binding fragment thereof according to any one of claims 1 to 11, comprising the steps of:
incubating the host cell according to claim 14 under culture conditions suitable for the expression of the improved amyloid protein oligomer-specific antibody or antigen-binding fragment thereof, and optionally, isolating and purifying the obtained product.

17. Use of the improved amyloid protein oligomer-specific antibody or antigen-binding fragment thereof according to any one of claims 1 to 11, the nucleic acid molecule according to claim 12, the expression vector according to claim 13, the host cell according to claim 14 or the composition according to claim 15 in the manufacture of a medicament for inhibiting Aβ aggregation and/or Aβ oligomer-induced cytotoxicity in a subject, or for treating and/or preventing a neurodegenerative disease in a subject, or for diagnosing whether a subject suffers from a neurodegenerative disease.

18. The improved amyloid protein oligomer-specific antibody or antigen-binding fragment thereof according to any one of claims 1 to 11, the nucleic acid molecule according to claim 12, the expression vector according to claim 13, the host cell according to claim 14 or the composition according to claim 15, for use in inhibiting Aβ aggregation and/or Aβ oligomer-induced cytotoxicity in a subject, or in treating and/or preventing a neurodegenerative disease in a subject, or in diagnosing whether a subject suffers from a neurodegenerative disease.

19. A method for inhibiting Aβ aggregation and/or Aβ oligomer-induced cytotoxicity in a subject, or treating and/or preventing a neurodegenerative disease in a subject, or for diagnosing whether a subject suffers from a neurodegenerative disease, comprising a step of administering to the subject or the subject's cells a therapeutically effective amount of the improved amyloid protein oligomer-specific antibody or antigen-binding fragment thereof according to any one of claims 1 to 11, the nucleic acid molecule according to claim 12, the expression vector according to claim 13, the host cell according to claim 14, or the composition according to claim 15.

20. The use according to claim 17, the improved amyloid protein oligomer-specific antibody or antigen-binding fragment thereof, the nucleic acid molecule, the expression vector, the host cell or the composition according to claim 18, or the method according to claim 19, wherein the neurodegenerative disease is selected from the group consisting of Alzheimer's disease, Parkinson's disease, Huntington's disease, amyotrophic lateral sclerosis, frontotemporal dementia and spinocerebellar ataxia, preferably, the neurodegenerative disease is selected from the group consisting of Alzheimer's disease, Parkinson's disease, Huntington's disease and amyotrophic lateral sclerosis.

21. Use of the improved amyloid protein oligomer-specific antibody or antigen-binding fragment thereof according to any one of claims 1 to 11, the nucleic acid molecule according to claim 12, the expression vector according to claim 13, the host cell according to claim 14 or the composition according to claim 15 in the manufacture of an agent for diagnosing the presence and/or level of toxic forms of amyloid in a sample from a subject.

22. Use of the improved amyloid protein oligomer-specific antibody or antigen-binding fragment thereof according to any one of claims 1 to 11, the nucleic acid molecule according to claim 12, the expression vector according to claim 13, the host cell according to claim 14 or the composition according to claim 15 in the manufacture of a medicament for specifically binding to the highly toxic amyloid protein oligomer Aβo*3F and inhibiting its neurotoxicity in a subject, wherein the highly toxic amyloid protein oligomer Aβo*3F is separated from an Aβ oligomer mixture by immunoprecipitation with the antibody 3F, typically characterized as an Aβ oligomer with high molecular weight, with a molecular weight of approximately 588 kDa and a diameter of approximately 10 nm based on size exclusion chromatography (SEC) analysis.
